# EUROPEAN PATENT APPLICATION

(11) **EP 1 662 257 A1**
(43) Date of publication of application: **31.05.2006**
(21) Application number: 04771753.3
(22) Date of filing: 11.08.2004
(51) Int. Cl.: G01N 33/543

(54) **TOOL FOR MEASURING OBJECT TO BE MEASURED, MEASURING DEVICE, AND MEASURING METHOD**

(30) Priority: 11.08.2003 JP 2003207184
(71) Applicant: KYOWA MEDEX CO., LTD., Chuo-ku, Tokyo 104-6004 (JP)
(72) Inventor: MIIKE, Akira, c/o Kyowa Medex Co., Ltd., Chuo-ku, Tokyo 104-6004 (JP); TSUNODA, Haruki, c/o Kyowa Medex Co., Ltd., Sunto-gun, Shizuoka 411-0932 (JP)
(74) Representative: Harding, Charles Thomas
(86) International application number: PCT/JP2004/011792
(87) International publication number: WO 2005/015217

(57) **Abstract**

According to the present invention, a device for measuring an object to be measured in a sample which comprises a support, sample addition site (S) and a detection site (Q), said sample addition site and said detection site being on the support, said support allowing the object to be measured to move by the capillary flow of a developing liquid, said detection site having a piezoelectric vibrator sandwiched between two electrodes, said piezoelectric vibrator having a trapper A (c1) immobilized thereon, or an analogue of the object to be measured (c1') immobilized thereon, and said support further comprising a binder retaining site(BR) where a binder (b1) is retained therein so that it is movable by the capillary flow of the developing liquid. Furthermore, the present invention provides a method for quantitatively determining an object to be measured in a sample using the device.

## Description

### Technical Field

The present invention relates to a device, an apparatus and a method for measuring an object to be measured contained in analyte samples such as biological specimens, foods and soils.

### Background Art

As a method for measuring an object to be measured in a sample, a measuring method by immunochromatography may be illustrated. Although immunochromatography is classified into flow through type and lateral flow type on the basis of the measurement principles thereof, in recent years, lateral flow type immunochromatography has become predominant. In connection with the lateral flow type immunochromatography, methods for measuring an object to be measured in a sample in which, for example, an apparatus for immunochromatography is used which comprises a first part and a second part that are present on an identical plane and that can be communicated with each other by the capillary flow are reported (for example, see Japanese Published Unexamined Patent Application No.63865/89 and Japanese Published Unexamined Patent Application No.73592/98).

In these methods, the first part is a sample addition site which contains a tracer supported so as to be movable, the tracer comprising a ligand that is specific for a labeled object to be measured that is labeled with a label, colored liposome, colored polymer beads or the like; and the second part contains an immobilized binding agent and is a site for visually detecting the presence/absence of the insoluble particle marker, the binding agent being specific for the object to be measured and being present in an amount which allows the tracer bound in the second part to be visualized.

Furthermore, a method for measuring an object to be measured in a sample is also reported in which an apparatus for immunochromatography is used which contains a first part containing a colloidal particle-labeled substance impregnated and dried in the presence of a quasi-soluble protein, and a second part containing an immobilizing reagent which can bind to the object to be measured and the colloidal particle-labeled substance (for example, Japanese Published Unexamined Patent Application No.32169/89).

However, since any of these methods is a measuring method in which an object to be measured in a sample is visually detected, such a method has been used mainly in qualitative analyses or semiquantitative analyses, but never have been applied to quantitative analyses. Therefore, applicable scope of the measurement by conventional immunochromatography has been restricted to a very limited scope such as assessment of pregnancy (for example, see, Japanese Published Unexamined Patent Application No.503174/89).

On the other hand, quantitative and qualitative analyses using a piezoelectric vibrator such as a quartz oscillator have been known. In this method, a complex is formed on the quartz oscillator through utilizing an interaction between antigen-antibody or an interaction between complementary nucleic acids, and alteration in the weight accompanied by formation of the complex is considered as an alteration in frequency of the quartz oscillator. Hitherto, for example, a method of detecting IgG in a sample using protein A immobilized on a quartz oscillator (for example, see Analytical Chemistry, American Chemical Society, 1987, No. 59, Vol. 23, pp. 2760-2763), a method of detecting a target nucleic acid in a sample using a probe DNA immobilized on a quartz oscillator (for example, see Japanese Published Unexamined Patent Application No.35269/89), and the like were reported.

In recent years, point of care testing (POCT) in which from sample collection to output of the measurement results is conducted in the vicinity of the test subject has been extensively carried out to comply with acceleration and diversification of the test processes, and thus, development of a measurement apparatus and a measuring method enabling quantitative analyses which can be applied to POCT has been desired.

### Disclosure of the Invention

An object of the present invention is to provide a device, an apparatus and a method for quantitatively analyzing an object to be measured included in a sample such as a biological specimen in a quick and simple manner with high accuracy.

The present invention relates to the following (1) to (35).
(1) A device for measuring an object to be measured in a sample which comprises a support, sample addition site (S) and a detection site (Q), said sample addition site and said detection site being on the support,
   said support allowing the object to be measured to move by the capillary flow of a developing liquid,
   said detection site having a piezoelectric vibrator sandwiched between two electrodes,
   said piezoelectric vibrator having a trapper A (c1) immobilized thereon, or an analogue of the object to be measured (c1') immobilized thereon, and
   said support further comprising a binder retaining site(BR) where a binder (b1) is retained therein so that it is movable by the capillary flow of the developing liquid.
(2) The device according to (1) described above
   wherein the binder retaining site is provided between the sample addition site and the detection site.
(3) The device according to (1) described above
   wherein the binder retaining site is provided on the opposite side of the sample addition site with the detection site being interposed therebetween.
(4) A device for measuring an object to be measured in a sample which comprises a support, sample addition site (S) and a detection site (Q), said sample addition site and said detection site being on the support,
   said support allowing the object to be measured to move by the capillary flow of a developing liquid,
   said detection site having a piezoelectric vibrator sandwiched between two electrodes,
   said piezoelectric vibrator having a trapper A (c1) immobilized thereon that binds to the object to be measured, or a trapper B (c3) immobilized thereon that binds to a complex of a binder with a labeled analogue of the object to be measured or the object to be measured and to a complex of a labeled binder with the object to be measured or the analogue of the object to be measured,
   said support further comprising a binder retaining site (BR) where the binder (b1) is retained so that it is movable by the capillary flow of the developing liquid, and
   said support further comprising an analogue of the object to be measured immobilizing site (DF) where the analogue of the object to be measured (b5) is immobilized between the binder retaining site and the detection site so that it is not moved by the capillary flow of the developing liquid, or an analogue of the object to be measured retaining site (DR) where the analogue of the object to be measured (b5) is retained on the support so that it is movable by the capillary flow of the developing liquid.
(5) The device according to any one of (1) to (4) described above wherein the binder is labeled.
(6) A device for measuring an object to be measured in a sample which comprises a support, sample addition site (S) and a detection site (Q), said sample addition site and said detection site being on the support,
   said support allowing the object to be measured to move by the capillary flow of a developing liquid,
   said detection site having a piezoelectric vibrator sandwiched between two electrodes, the piezoelectric vibrator having a binder (c2) immobilized thereon, and
   said support further comprising an analogue of the object to be measured retaining site (DR) where the analogue of the object to be measured (b2) is retained so that it is movable by the capillary flow of the developing liquid.
(7) The device according to (6) described above
   wherein the analogue of the object to be measured retaining site is provided between the sample addition site and the detection site.
(8) The device according to (6) described above
   wherein the analogue of the object to be measured retaining site is provided on the opposite side of the sample addition site with the detection site being interposed therebetween.
(9) A device for measuring an object to be measured in a sample which comprises a support, sample addition site (S) and a detection site (Q), said sample addition site and said detection site being on the support,
   said support allowing the object to be measured to move by the capillary flow of a developing liquid,
   said detection site having a piezoelectric vibrator sandwiched between two electrodes, the piezoelectric vibrator having a trapper B (c3) immobilized thereon, and
   said support further comprising an analogue of the object to be measured retaining site (DR) where an analogue of the object to be measured (b2) is retained so that it is movable by the capillary flow of the developing liquid, and a binder retaining site (BR) where a binder (b3) is retained so that it is movable by the capillary flow of the developing liquid.
(10) A device for measuring an object to be measured in a sample which comprises a support, sample addition site (S) and a detection site (Q), said sample addition site and said detection site being on the support,
   said support allowing the object to be measured to move by the capillary flow of a developing liquid,
   said detection site having a piezoelectric vibrator sandwiched between two electrodes, the piezoelectric vibrator having a binder (c2) immobilized thereon that binds to the object to be measured,
   said support further comprising an analogue of the object to be measured retaining site (DR) where an analogue of the object to be measured (b2) is retained so that it is movable by the capillary flow of the developing liquid, and
   said support further comprising a binder immobilizing site (BF) where a binder (b4) is immobilized on the support between the analogue of the object to be measured retaining site and the detection site so that it is not movable by the capillary flow of the developing liquid.
(11) The device according to any one of (6) to (10) described above wherein the analogue of the object to be measured is labeled.
(12) The device according to any one of (5) or (11) described above wherein the label is an insoluble particle.
(13) The device according to (12) described above
   wherein the insoluble particle is a metal colloid or latex.
(14) A device for measuring an object to be measured in a sample which comprises a support, sample addition site (S) and a detection site (Q), said sample addition site and said detection site being on the support,
   said support allowing the object to be measured to move by the capillary flow of a developing liquid,
   said detection site having a piezoelectric vibrator sandwiched between two electrodes, and
   said piezoelectric vibrator having a trapper A (c1), an analogue of the object to be measured (c1'), a binder (c2) or a trapper B (c3).
(15) The device according to any one of (1) to (14) described above which further comprises a developer absorbing site (d).
(16) The device according to any one of (1) to (15) described above wherein the detection site has another piezoelectric vibrator which is sandwiched between two electrodes and on which none of the trapper A (c1), the analogue of the object to be measured (c1'), the binder (c2) and the trapper B (c3) are immobilized, in addition to the piezoelectric vibrator which is sandwiched between two electrodes and on which the trapper A (c1), the analogue of the object to be measured (c1'), the binder (c2) or the trapper B (c3) is immobilized.
(17) The device according to any one of (1) to (16) described above wherein the piezoelectric vibrator is a quartz oscillator.
(18) An apparatus for measuring an object to be measured which comprises the device according to any one of claims 1 to 17, a frequency measuring circuit which measures the frequency of vibration of the piezoelectric vibrator and which is connected to the electrode of the piezoelectric vibrator of the device, and a concentration arithmetic circuit which calculates a concentration of the object to be measured based on the frequency and which is connected to the frequency measuring circuit.
(19) A method for quantitatively determining an object to be measured in a sample which comprises the steps of:
   preparing a piezoelectric vibrator having a substance immobilized thereon, said substance being a trapper A (c1) or an analogue of the object to be measured (c1'),
   supplying the sample and a binder to the substance through a support where the object to be measured and the binder are movable by the capillary flow of the developing liquid,
   allowing the object to be measured in the sample to bind specifically to the substance immobilized on the piezoelectric vibrator, and
   quantitatively determining alteration in frequency of a piezoelectric vibrator generated by the specific binding with the substance immobilized on the piezoelectric vibrator.
(20) The method of the quantitative determination according to (19) described above wherein the sample and the binder are supplied by the capillary flow in the same direction toward the trapper A or the analogue of the object to be measured.
(21) The method of the quantitative determination according to (19) described above wherein the sample and the binder are supplied by the capillary flow in the reverse direction with respect to the trapper A or the analogue of the object to be measured.
(22) A method for quantitatively determining an object to be measured in a sample which comprises the steps of:
   preparing a piezoelectric vibrator having a substance immobilized thereon, said substance being a trapper A (c1) or a trapper B (c3),
   supplying the sample and a binder to the substance through a support where the object to be measured and the binder are movable by the capillary flow of the developing liquid, but where an analogue of the object to be measured (b5) is immobilized so that it is not movable by the capillary flow of the developing liquid,
   allowing the object to be measured in the sample to bind specifically to the substance immobilized on the piezoelectric vibrator, and
   quantitatively determining alteration in frequency of a piezoelectric vibrator generated by the specific binding with the substance immobilized on the piezoelectric vibrator.
(23) A method for quantitatively determining an object to be measured in a sample which comprises the steps of:
   preparing a piezoelectric vibrator having a substance immobilized thereon, said substance being a trapper A (c1) or an analogue of the object to be measured (c1'),
   supplying the sample and a binder to the substance through a support where the object to be measured and the binder are movable by the capillary flow of the developing liquid,
   allowing the object to be measured in the sample to bind specifically to the substance immobilized on the piezoelectric vibrator, and
   quantitatively determining alteration in frequency of a piezoelectric vibrator generated by the specific binding with the substance immobilized on the piezoelectric vibrator.
(24) The method according to any one of (17) to (23) described above wherein the binder is labeled.
(25) A method for quantitatively determining an object to be measured in a sample which comprises the steps of:
   preparing a piezoelectric vibrator having a substance immobilized thereon, said substance being a binder (c2),
   supplying the sample and an analogue of the object to be measured to the substance through a support where the object to be measured and the analogue of the object to be measured are movable by the capillary flow of the developing liquid,
   allowing the object to be measured in the sample to bind specifically to the substance immobilized on the piezoelectric vibrator, and
   quantitatively determining alteration in frequency of a piezoelectric vibrator generated by the specific binding with the substance immobilized on the piezoelectric vibrator.
(26) The method according to (25) described above
   wherein the sample and the analogue of the object to be measured are supplied by the capillary flow in the same direction toward the binder.
(27) The method according to (25) described above
   wherein the sample and the analogue of the object to be measured are supplied by the capillary flow in the reverse direction with respect to the binder.
(28) A method for quantitatively determining an object to be measured in a sample which comprises the steps of:
   preparing a piezoelectric vibrator having a substance immobilized thereon, said substance being a trapper B (c3),
   supplying the sample, an analogue of the object to be measured (b2) and a binder (c3) to the substance through a support where the object to be measured, the analogue of the object to be measured (b2) and the binder (c3) are movable by the capillary flow of the developing liquid,
   allowing the object to be measured in the sample to bind specifically to the substance immobilized on the piezoelectric vibrator, and
   quantitatively determining alteration in frequency of a piezoelectric vibrator generated by the specific binding with the substance immobilized on the piezoelectric vibrator.
(29) A method for quantitatively determining an object to be measured in a sample which comprises the steps of:
   preparing a piezoelectric vibrator having a substance immobilized thereon, said substance being a binder (c2),
   supplying the sample and an analogue of the object to be measured to the substance through a support where the object to be measured and the analogue of the object to be measured are movable by the capillary flow of the developing liquid, but where a binder (b4) is immobilized so that it is not movable by the capillary flow of the developing liquid,
   allowing the object to be measured in the sample to bind specifically to the substance immobilized on the piezoelectric vibrator, and
   quantitatively determining alteration in frequency of a piezoelectric vibrator generated by the specific binding with the substance immobilized on the piezoelectric vibrator.
(30) The method according to any one of (25) to (29) described above wherein the analogue of the object to be measured is a labeled substance.
(31) The method according to (24) or (30) described above wherein the label is an insoluble particle.
(32) The method according to (31) described above
   wherein the insoluble particle is a metal colloid or latex.
(33) A method for quantitatively determining an object to be measured in a sample which comprises the steps of:
   preparing a piezoelectric vibrator having a substance immobilized thereon, said substance being a trapper A (c1),
   supplying the sample to the substance through a support where the object to be measured is movable by the capillary flow of the developing liquid,
   allowing the object to be measured in the sample to bind specifically to the substance immobilized on the piezoelectric vibrator, and
   quantitatively determining alteration in frequency of a piezoelectric vibrator generated by the specific binding with the substance immobilized on the piezoelectric vibrator.
(34) The method according to any one of (17) to (29) described above wherein a second piezoelectric vibrator on which none of the trapper A (c1), the analogue of the object to be measured (c1'), the binder (c2) and the trapper B (c3) are immobilized is used, the method further comprising supplying the sample to a first piezoelectric vibrator on which the trapper A (c1), the analogue of the object to be measured (c1'), the binder (c2) or the trapper B (c3) is immobilized and to the second piezoelectric vibrator, and using the second frequency as a control.
(35) The method according to any one of (19) to (34) described above wherein the piezoelectric vibrator is a quartz oscillator.

The device for measuring an object to be measured in a sample and the method for measuring an object to be measured in a sample according to the present invention are characterized in that in a device for immunochromatography and a method using the device for immunochromatography, a piezoelectric vibrator and a method of the detection using the same are utilized as the detection device and the detection method, respectively. The present invention comprises allowing a sample added to a sample addition site provided on a support to move on the support by the capillary flow of the developing liquid, finally forming a complex on a piezoelectric vibrator positioned at a detection site, and detecting the amount of the formed complex in terms of alteration in the frequency.

The sample which may be used in the present invention is not particularly limited, but examples thereof include biological specimens such as whole blood, plasma, serum, cord blood, spinal fluid, saliva, amniotic fluid, urine, sweat, pancreatic fluid and eye drop, foods, soils and the like. Also, a diluent obtained by adding an aqueous medium, an organic solvent, a mixed solvent of the aqueous medium and the organic solvent to any one of these biological specimens, can be used as a sample.

The aqueous medium is not particularly limited as long as it dissolves the biological specimen, but examples thereof include deionized water, distilled water, a buffer solution and the like. Among them, a buffer solution is preferred. A buffer use in the buffer solution is not particularly limited as long as it has a buffer action, but examples thereof include, a lactate buffer, a citrate buffer , a acetate buffer, a succinate buffer, a phthalate buffer, a phosphate buffer, a triethanolamine buffer, a diethanolamine buffer , a lysine buffer, a barbiturate buffer, a tris(hydroxymethyl)- aminomethane buffer, an imidazole buffer, a malate buffer, an oxalate buffer, a glycine buffer, a borate buffer, a carbonatebuffer, a glycine buffer , a Good's buffer and the like having a pH of 1 to 11. Examples of the Good's buffer include, 2-morpholinoethanesulfonic acid (MES), bis(2-hydroxyethyl)imino tris(hydroxymethyl)methane(Bis-Tris), N-(2-acetoamide)imino diacetic acid (ADA), piperazine-N,N'-bis(2-ethanesulfonic acid) (PIPES), N-(2-acetoamide)-2-aminoethanesulfonic acid (ACES), 3-morpholino-2-hydroxypropanesulfonic acid (MOPSO), N,N-bis(2-hydroxyethyl)-2-aminoethanesulfonic acid (BES), 3-morpholinopropanesulfonic acid (MOPS), N-[tris-(hydroxymethyl) methyl]-2-aminoethanesulfonic acid (TES), 2-[4-(2-hydroxyethyl)-l-piperazinyl]ethanesulfonic acid (HEPES), 3-[N,N-bis(2-hydroxyethyl)amino]-2-hydroxypropanesulfonic acid (DIPSO), N-[tris(hydroxymethyl)-methyl]-2-hydroxy-3-aminopropanesulfonic acid (TAPSO), piperazine-N,N'-bis(2-hydroxypropanesulfonic acid) (POPSO), 3-[4-(2-hydroxyethyl)-1-piperazinyl]-2-hydroxypropanesulfonic acid (HEPPSO), 3-[4-(2-hydroxyethyl)-1-piperazinyl]propanesulfonic acid [(H)EPPS], N-[tris(hydroxymethyl)methyl]glycine (Tricine), N,N-bis(2-hydroxyethyl)glycine (Bicine), N-tris(hydroxymethyl)-methyl-3-aminopropanesulfonic acid (TAPS), N-cyclohexyl-2-aminoethanesulfonic acid (CHES), N-cyclohexyl-3-amino-2-hydroxypropanesulfonic acid (CAPSO), N-cyclohexyl-3-aminopropanesulfonic acid (CAPS) and the like. Concentration of the buffer is not particularly limited as long as it is a concentration suitable for the measurement, but is preferably 0.001 to 2.0 mol/L, more preferably 0.005 to 1.0 mol/L, and particularly preferably 0.01 to 0.1 mol/L.

Examples of the organic solvent include acetonitrile, hexane, methanol, ethanol, dichloromethane, chloroform, acetone, dimethylformamide, dioxane, dimethylsulfoxide and the like.

Furthermore, food and soil subjected to a pretreatment may be used as a sample. Herein, the pretreatment of the food and the soil may be for example, extraction of the component in the food or the soil with a suitable solvent such as the aforementioned aqueous medium, the aforementioned organic solvent, or the aforementioned mixture of the aqueous medium and the organic solvent; chemical modification and the like.

The chemical modification may be for example, structural conversion of the component in the food or the soil by a chemical reagent, and the like.

The object to be measured according to the present invention is not particularly limited as long as it specifically forms a complex with a certain substance, and examples thereof include components which can be measured with an enzyme reaction, components which can be measured with an antigen-antibody reaction, components which can be measured by other specific reaction, and the like.

Examples of the biological component which may be measured with the enzyme reaction include, glucose, 1,5-anhydroglucitol, glycoalbumin, hemoglobin A1c, fucose, urea, uric acid, ammonia, creatinine, total cholesterol, free cholesterol, cholesterol in high density lipoprotein (HDL-C), cholesterol in low density lipoprotein (LDL-C), cholesterol in very low density lipoprotein (VLDL-C), cholesterol in remnant-like lipoprotein (RLP-C), triglyceride, phospholipid, total protein, albumin, globulin, bilirubin, bile acid, sialic acid, lactic acid, pyruvic acid, free fatty acid, ceruloplasmin, alanine aminotransferase (ALT), aspartate aminotransferase (AST), creatine phosphokinase (CPK), phosphokinase (PK), amylase, lipase, choline esterase, γ-glutamyl transpeptidase, leucine aminopeptidase, L-lactate dehydrogenase (LDH), aldolase, alkaline phosphatase, acid phosphatase, N-acetylglucosaminidase, guanase, monoamine oxydase and the like.

Examples of the component which may be measured with the antigen-antibody reaction include, IgG, IgM, IgA, IgE, apoprotein AI, apoprotein AII, apoprotein B, apoprotein E, rheumatoid factor, D-dimer, oxidized LDL, glycated LDL, glycoalbumin, triiodothyronine (T3), total thyroxine (T4), drugs (antiepileptic drug and the like), C-reactive proteins (CRP), cytokines, α-fetoprotein (AFP), cancer embryonic antigen (CEA), CA19-9 (carbohydrate antigen 19-9), CA15-3 (carbohydrate antigen 15-3), CA-125 (carbohydrate antigen 125), PIVKA-II (Protein induced by vitamin K absence-II), parathyroid hormone (PTH), human chorionic gonadotrophin (hCG), thyroid stimulating hormone (TSH), insulin, C-peptide, estrogen, anti-glutamic acid decarboxylase (GAD) antibodies, pepsinogen, HBV antigen, anti-hepatitis B virus (HBV) antibodies, hepatitis C viral (HCV) antigen, anti-HCV antibodies, adult T-cell leukaemia virus type 1 (HTLV-I) antigen, anti-HTLV-I antibodies, human immune deficiency virus (HIV) antibodies, influenzae viral antigen, anti-influenzae virus antibodies, anti-tubercle bacilli antibodies, tubercle bacilli antigen (TBGL) mycoplasma antibodies, hemoglobin A1c, atrial natriuretic peptide (ANP), brain natriuretic peptide (BNP), troponin T, troponin I, creatinine kinase-MB (CK-MB), myoglobin, H-FABP (fatty acid binding protein derived from human heart), mycotoxins [deoxynivalenol (DON), nivalenol (NIV), T-2 toxin (T2) and the like], endocrine disrupting substances [bisphenol A, nonylphenol, dibutyl phthalate, polychlorinated biphenyls (PCB), dioxins, p,p'-dichlorodiphenyltrichloroethane, tributyltin and the like], bacteria such as *Escherichia coli,* food allergic substances of egg, milk, wheat, buckwheat, peanut and the like, allergic substances of mites such as Deratophagoides farinae and Dermatophagoides teronyssinus, anti-allergic substance antibodies and the like.

Examples of components which may be measured with other specific binding include nucleic acids, lectins and the like, and specific examples thereof include, DNAs or RNAs encoding oncogenes such as ras, cancer suppressor genes such as p53, peptide, nucleic acids, aptamers, glycoproteins and the like.

The device for measuring an object to be measured according to the present invention comprises a sample addition site (S), a support (e) and a detection site (Q), said sample addition site, said support and said detection site being on a base material, and optionally further comprises a developing liquid addition site (B), a developing liquid absorbing site (d) and the like.

The support (e) in the present invention is not particularly limited as long as it permits communication between the sample addition site (S), the detection site (Q) and optionally providing the developing liquid addition site (B) by the capillary flow of the developing liquid. The developing liquid addition site is the site where a developing liquid is added which contains or does not contain the binder, the labeled binder, the analogue of the object to be measured, the labeled analogue of the object to be measured or the like, it also allows the object to be measured in the sample to move between the sample addition site, the detection site and optionally providing the developing liquid addition site by the capillary flow of the developing liquid, and it further allows the labeled binder, the labeled analogue of the object to be measured, the binder or the analogue of the object to be measured, to immobilize or to retain so that it is movable by the developing liquid.

The materials of the sample addition site (S) and the support (e) may be the same or different, and examples thereof include glassfiber, cellulose, nylon, crosslinked dextran, various paper for chromatography, nitrocellulose and the like. Among them, nitrocellulose is preferred.

Examples of the developing liquid include the aforementioned aqueous media, organic solvents and the like.

In the present invention, the binder which is retained on the support so as to be movable together with the developing liquid or which is immobilized on the support or on the crystal oscillator, is not particularly limited as long as it specifically binds to the object to be measured, and the examples include, either one element of combination consisting of an antigen and an antibody or an aptamer that specifically bind to the antigen, either one element of combination consisting of a saccharide and lectin to the saccharide, either one element of combination of a DNA and a complementary DNA to the DNA, and the like.

In the present invention, the analogue of the object to be measured which is immobilized on the support or the piezoelectric vibrator or the analogue of the object to be measured in the labeled analogue of the object to be measured which is retained on the support so as to be movable together with the developing liquid, is not particularly limited as long as it reacts with the binder competitively to the object to be measured in the sample, and examples thereof include, the object to be measured itself, any substance containing an epitope to the binder, and the like.

The label to be bound to the binder or the analogue of the object to be measured in the present invention is not particularly limited as long as the complex produced on the piezoelectric vibrator can be detected based on the amount of alteration in the frequency, but a label having a great mass is preferred. Examples of the label having a great mass include insoluble particles and the like. Examples of the insoluble particle include metal colloids, latex and the like. Examples of the metal colloid include gold colloids and the like. Particle size of the gold colloid is preferably 1 to 500 nm, and more preferably 10 to 100 nm. Particle size of the latex is preferably 1 to 2,000 nm, and more preferably 50 to 500 nm.

The labeled binder retained on the support so as to be movable together with the developing liquid in the present invention is not particularly limited as long as it specifically binds to the object to be measured, and transmits the information depending on the amount of the complex including the labeled binder produced on the piezoelectric vibrator. The labeled analogue of the object to be measured retained on the support so as to be movable together with the developing liquid in the present invention is not particularly limited as long as it specifically binds to the binder, and transmits the information depending on the amount of the complex containing the labeled analogue of the object to be measured produced on the piezoelectric vibrator.

The binder or the analogue of the object to be measured and the label may be bound either physically or chemically. Examples of the physical bond include physical adsorption and the like. Examples of the chemical bond include covalent bonds, noncovalent bonds and the like. Examples of the noncovalent bond include electrostatic bonds, hydrogen bonds, hydrophobic bonds, coordinate bonds and the like. As the process for preparing the labeled binder by binding the label and the binder or the analogue of the object to be measured via a covalent bond, for example, a process which binds the label and the binder via a crosslinking agent using a crosslinking agent such as a divalent crosslinking agent or the like may be illustrated.

According to the present invention, the piezoelectric vibrator which may be used in the detection site of the object to be measured in the sample is not particularly limited as long as it consists of a crystal having a piezoelectric effect, and examples of the crystal include, crystals such as crystalline quartz, Rochelle salt and electronic ore, oxide single crystals such as lithium tantalate (LiTaO₂) and lithium niobate (LiNbO₃), zinc oxide (ZnO), and the like. Among them, crystalline quartz is preferred.

The piezoelectric vibrator is preferably designed such that it is connected to an adequate frequency measuring circuit to detect frequency of the piezoelectric vibrator by a display or the like. More preferably, it is connected to a concentration arithmetic circuit such that an arithmetical operation of the concentration of the object to be measured is performed based on alteration in detected frequency. The piezoelectric vibrator is not particularly limited as long as it is a piezoelectric vibrator sandwiched between the electrodes. As the quartz oscillator, a sensor chip consisting of a quartz oscillator manufactured by for example, Seiko · Tokyo Denpa Co., Ltd. as well as Initium, Inc. may be used.

The device of the present invention has a first piezoelectric vibrator on which the binder, the trapper A, the trapper B or the analogue of the object to be measured is immobilized, however, the device of the present invention preferably has, in addition to the first piezoelectric vibrator, a second piezoelectric vibrator on which any of the binder, the trapper A, the trapper B or the analogue of the object to be measured is not immobilized.

Any complex which is formed by specific binding to the binder, the trapper A, the trapper B or the analogue of the object to be measured on the piezoelectric vibrator is permitted, and examples thereof include complexes produced by a sandwich process, complexes produced by a competitive process, and the like. Examples of specific mode of the complex include complexes having a constitution of "trapper A-object to be measured-labeled binder", as well as "analogue of the object to be measured-labeled binder", "binder-labeled analogue of the object to be measured", "trapper B-binder-labeled analogue of the object to be measured", "trapper B-labeled binder-object to be measured", "trapper A-object to be measured" and the like.

The trapper A which may be immobilized on the piezoelectric vibrator in the present invention is not particularly limited as long as it binds to the object to be measured, and thus can produce a sandwich type complex (trapper A-object to be measured-labeled binder) per se, by a sandwich reaction in which the object to be measured and the labeled binder are involved. The recognition site of the object to be measured in the labeled binder and the recognition site of the object to be measured in the trapper A may be the same, but are preferably different each other. Examples of the trapper A include, antibodies that bind to the antigen as the object to be measured, substances or single strand DNAs that specifically bind to a double strand DNA obtained by binding of a single strand DNA, as the object to be measured, and a labeled DNA that is complementary thereto, and the like.

The trapper B which may be immobilized on the piezoelectric vibrator in the present invention is not particularly limited as long as it binds to a complex of the binder with the labeled analogue of the object to be measured or the object to be measured, and a complex of the labeled binder with the object to be measured or the analogue of the object to be measured, but does not produce a sandwich type complex that sandwiches the object to be measured in between. Examples of the trapper B include antibodies that recognize an Fc region of the antibody that binds to the antigen as the object to be measured.

Immobilization of the binder, the trapper A, the trapper B or the analogue of the object to be measured on the piezoelectric vibrator is not particularly limited as long as immobilization is perfected such that the trapper A, the trapper B or the analogue of the object to be measured is not moved when the liquid which has migrated by the capillary flow of the developing liquid on the support passes through the piezoelectric vibrator. The immobilization may be based on either physical or chemical bond. Examples of the physical bond include physical adsorption and the like, while examples of the chemical bond include covalent bonds, hydrogen bonds, hydrophobic bonds, coordinate bonds and the like. Specifically, a process of the immobilization by allowing a sulfhydrylated trapper A, a sulfhydrylated trapper B or a sulfhydrylated analogue of the object to be measured to be bound to a gold electrode on the piezoelectric vibrator may be illustrated.

In the present invention, a site where the binder or the labeled binder or the analogue of the object to be measured or the labeled analogue of the object to be measured is retained can be provided on the support as a developing liquid addition site. The material which may be used for the developing liquid addition site is not particularly limited as long as it can retain or immobilize such that the object to be measured in the sample becomes movable between the sample addition site and the support by the capillary flow of the developing liquid, and further, if necessary, such that the labeled binder, the labeled analogue of the object to be measured, the binder and the analogue of the object to be measured become movable together with the developing liquid.

The material of the developing liquid addition site may be the same as or different from the material of the sample addition site and the support, and examples thereof include, glassfiber, cellulose, nylon, crosslinked dextran, various paper for chromatography, nitrocellulose and the like. Among them, nitrocellulose is preferred.

In the present invention, for the purpose of absorbing and retaining the developing liquid containing components not captured by the binder, the trapper A, the trapper B or the analogue of the object to be measured which has been immobilized on the piezoelectric vibrator, a developing liquid absorbing site (d) can be provided on the support. A water absorptive high molecular compound can be used for the developing liquid absorbing site. Examples of the water absorptive high molecular compound include cellulose, glassfiber, cotton, polyurethane and the like.

The frequency measuring circuit is not particularly limited, but any known one can be used as long as it can be used by connecting to two electrodes of the device for measuring the object to be measured of the present invention, and can detect the frequency of the piezoelectric vibrator. The frequency measuring circuit preferably has a display member for displaying the frequency, but the frequency measuring circuit not having a display member is also permitted. The concentration arithmetic circuit is not particularly limited, but any known one can be used as long as it can be used by connecting to the frequency measuring circuit, and can calculate the concentration from the frequency obtained with the sample having unknown concentration on the basis of the data of frequency obtained using an authentic standard having known concentration. The concentration arithmetic circuit preferably has a display member for displaying the concentration, but the concentration arithmetic circuit not having a display member is also permitted.

The frequency measuring circuit and the concentration arithmetic circuit are preferably integrated and incorporated into the device for measuring an object to be measured of the present invention, but the frequency measuring circuit or the frequency measuring circuit connected to the concentration arithmetic circuit may be constituted as separate parts.

Examples of the frequency measuring circuit and the concentration arithmetic circuit include a thermoregulated measuring part and a data processing apparatus used in AFFINIX Q manufactured by Initium, Inc., and the like. It is preferred that a display panel for showing the results is incorporated in the arithmetic circuit.

For the measurement in which an authentic standard having a known concentration is used for producing a calibration curve, the identical apparatus for measuring the object to be measured may be used to execute in a time series manner. However, it is preferred that the measurement of the object to be measured in the sample and the measurement of the authentic sample are concomitantly carried out using an apparatus constituted with multiple apparatuses for measuring the object to be measured in combination. Furthermore, a method in which a device having two piezoelectric vibrators provided together on single support is used, wherein a first piezoelectric vibrator having the trapper A (c1), the analogue of the object to be measured (c1'), the binder (c2) or the trapper B (c3) is immobilized thereon is used as one piezoelectric vibrator, while a second piezoelectric vibrator having any one of the trapper A (c1), the analogue of the object to be measured (c1'), the binder (c2) and the trapper B (c3) is not immobilized thereon is used as another piezoelectric vibrator; and wherein correction is conducted upon determination of the concentration of the object to be measured in the sample on the basis of the alteration in the frequency of the first piezoelectric vibrator through subtracting the alteration in the frequency of the second piezoelectric vibrator is particularly preferred.

Reaction temperature employed when the object to be measured in the sample and the authentic standard are measured is not particularly limited as long as a it enables the specific reaction, but the reaction is conducted usually at 0 to 100°C, preferably 10 to 60°C, and more preferably 20 to 40°C.

A control line may be also included in the apparatus for measuring the object to be measured in the present invention for the purpose of discriminating definite addition of the sample. The control line which may be used is any one generally used in immunochromatography, and may be formed either on the support or on the piezoelectric vibrator. However, it is preferably formed on the support. Examples of the control line may include those of the following embodiments. When the sample is a biological specimen, exemplary control line may include a site on the support where a functional substance-binding secondary antibody is retained so as to be movable which has a functional substance such as an enzyme, a fluorescent substance or a luminescent substance bound to a secondary antibody which binds to a protein such as albumin, and a site on the support where a primary antibody that binds to a protein such as albumin included in the biological specimen is immobilized thereon. When the biological specimen is added as the sample without fail, the protein such as albumin in the sample will reach to the control line, and form a complex (complex including a primary antibody, a protein such as albumin and a labeled secondary antibody) on this control line by the sandwich reaction. Presence/absence of the addition of the sample can be verified by detecting the functional substance in this complex with, for example, a colorimetric method, a fluorescence method or a luminescent method.

Additionally, in case in which the labeled binder moves on the support in the same direction as that of the sample whereby forming a sandwich type complex on the piezoelectric vibrator, the exemplary control line may, for example, detect the labeled binder which has not been captured on the piezoelectric vibrator. Examples of such a control line include, control lines having a trapper, which binds to the labeled binder, immobilized on the support at an arbitrary position. When the sample is added without fail, the labeled binder which has not been captured by the piezoelectric vibrator is captured at this control line. When the labeled binder is captured at this control line, the control line is colorized by the label in the labeled binder (for example, gold colloidal particle, colored latex or the like), therefore, addition of the sample can be visually assessed. The control line is preferably provided on the support, for example, on the opposite side of the sample addition site with respect to the detection site.

Proportion of the object to be measured, the labeled analogue of the object to be measured and the binder is not particularly limited, but may be defined to be preferably 0 to 1,000,000:1:1 to 1,000,000, and more preferably 0 to 1,000:1:1 to 1,000.

Hereinafter, the apparatus for measuring the concentration of an object to be measured in a sample and the method of the measurement of the present invention will be specifically explained.

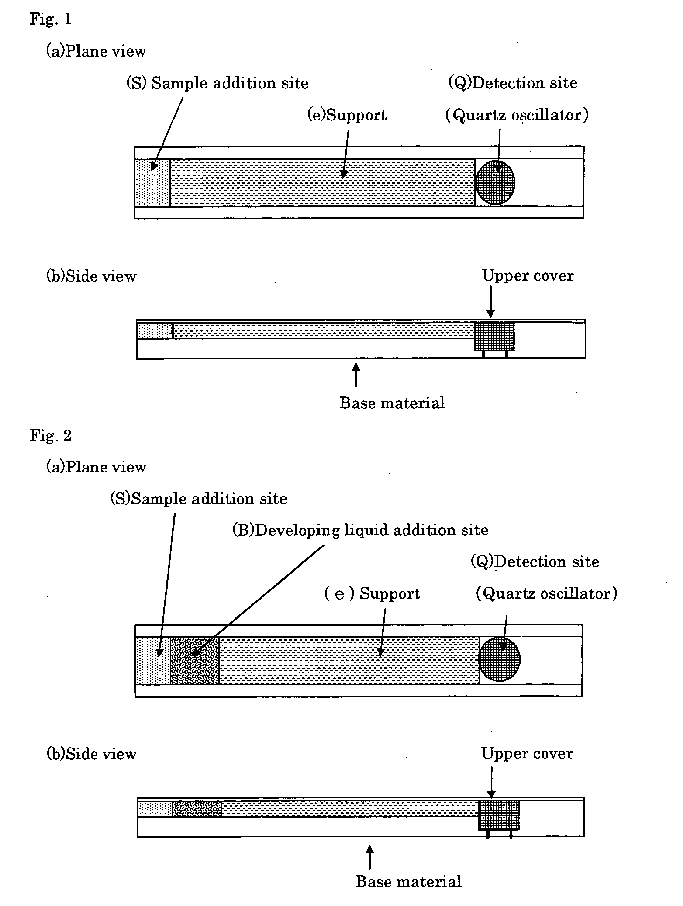

Fig. 1 is a schematic view illustrating one of specific embodiments of the device for measuring an object to be measured of the present invention: Fig. 1(a) shows the plane view; and Fig. 1(b) shows the side view.

This device comprises a sample addition site (S), a support (e) and a detection site (Q) having a quartz oscillator sandwiched between two electrodes which are provided on a base material; the sample addition site, the support and the detection site being covered by an upper cover and connected so as to be movable by the capillary flow of the developing liquid. On the quartz oscillator is immobilized a trapper A, an analogue of the object to be measured, a binder or a trapper B. For the sample addition site may be used a material that is the same as the support, but different one may be also used. At the sample addition site, the upper cover has a pore provided such that the sample can be added thereto. The material for the base material is not particularly limited, but a resin is preferably used. The two electrode of the quartz oscillator are provided to be connectable with the outside.

Fig. 2 is a schematic view illustrating one of specific embodiments of the device for measuring an object to be measured of the present invention: Fig. 2(a) shows the plane view; and Fig. 2(b) shows the side view.

This device comprises a sample addition site (S), a developing liquid addition site (B), a support (e) and a detection site (Q) having a quartz oscillator sandwiched between two electrodes which are provided on a base material; the sample addition site, the developing liquid addition site, the support and the detection site being covered by an upper cover and connected so as to be movable by the capillary flow of the developing liquid. For the sample addition site and the developing liquid addition site may be used a material that is the same as the support, but different one may be also used. At the sample addition site, the upper cover has a pore provided such that the sample can be added thereto. Further, at the developing liquid addition site, a pore may or may not be provided. Also, at the developing liquid addition site can be provided a binder immobilizing site where the binder is immobilized on the support so as not to be moved by the capillary flow of the developing liquid, a binder retaining site where the binder is retained so as to be movable by the capillary flow of the developing liquid, an analogue of the object to be measured immobilizing site where the analogue of the object to be measured is immobilized on the support so as not to be moved by the capillary flow of the developing liquid, or an analogue of the object to be measured retaining site where the analogue of the object to be measured is retained so as to be movable by the capillary flow of the developing liquid. The material for the base material is not particularly limited, but a resin is preferably used.

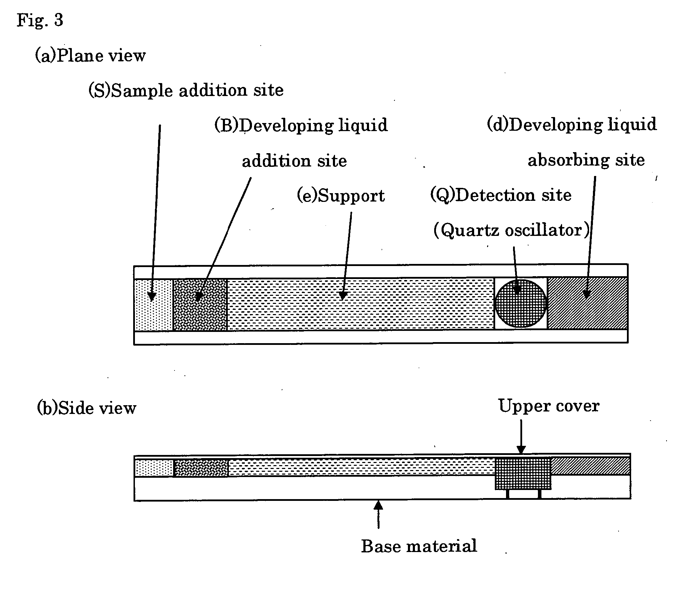

Fig. 3 is a schematic view illustrating one of specific embodiments of the device for measuring an object to be measured of the present invention: Fig. 3(a) shows the plane view; and Fig. 3(b) shows the side view.

This device comprises a sample addition site (S), a developing liquid addition site (B), a support (e), a detection site (Q) having a quartz oscillator sandwiched between two electrodes, and a developing liquid absorbing site (d) which are provided on a base material; the sample addition site, the developing liquid addition site, the support, the detection site and the developing liquid absorbing site being covered by an upper cover and connected so as to be movable by the capillary flow of the developing liquid. For the sample addition site and the developing liquid addition site may be used a material that is the same as the support, but different one may be also used. At the sample addition site, the upper cover has a pore provided such that the sample can be added thereto. At the developing liquid addition site, a pore may or may not be provided. Also, at the developing liquid addition site can be provided a binder immobilizing site where the binder is immobilized on the support so as not to be moved by the capillary flow of the developing liquid, a binder retaining site where the binder is retained so as to be movable by the capillary flow of the developing liquid, an analogue of the object to be measured immobilizing site where the analogue of the object to be measured is immobilized on the support so as not to be moved by the capillary flow of the developing liquid, or an analogue of the object to be measured retaining site where the analogue of the object to be measured is retained so as to be movable by the capillary flow of the developing liquid. The material for the base material is not particularly limited, but a resin is preferably used. For the developing liquid absorbing site can be used a water absorptive high molecular compound. Examples of the water absorptive high molecular compound include e.g., cellulose, glassfiber, cotton, polyurethane and the like.

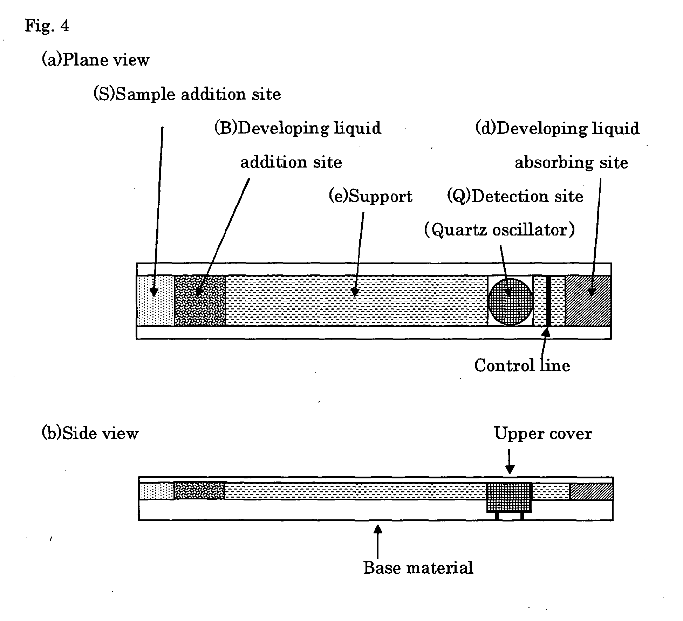

Fig. 4 is a schematic view illustrating one of specific embodiments of the device for measuring an object to be measured of the present invention: Fig. 4(a) shows the plane view; and Fig. 4(b) shows the side view.

This device comprises a sample addition site (S), a developing liquid addition site (B), a support (e), a detection site (Q) having a quartz oscillator sandwiched between two electrodes, a developing liquid absorbing site (d), and a control line which are provided on a base material; the sample addition site, the developing liquid addition site, the support, the detection site, the developing liquid absorbing site and a control line being covered by an upper cover and connected so as to be movable by the capillary flow of the developing liquid. For the sample addition site and the developing liquid addition site may be used a material that is the same as the support, but different one may be also used. Furthermore, at the sample addition site, the upper cover has a pore provided such that the sample can be added thereto. At the developing liquid addition site, a pore may or may not be provided. Also, at the developing liquid addition site can be provided a binder immobilizing site where the binder is immobilized on the support so as not to be moved by the capillary flow of the developing liquid, a binder retaining site where the binder is retained so as to be movable by the capillary flow of the developing liquid, an analogue of the object to be measured immobilizing site where the analogue of the object to be measured is immobilized on the support so as not to be moved by the capillary flow of the developing liquid, or an analogue of the object to be measured retaining site where the analogue of the object to be measured is retained so as to be movable by the capillary flow of the developing liquid. The material for the base material is not particularly limited, but a resin is preferably used. For the developing liquid absorbing site can be used a water absorptive high molecular compound. Examples of the water absorptive high molecular compound include, cellulose, glassfiber, cotton, polyurethane and the like.

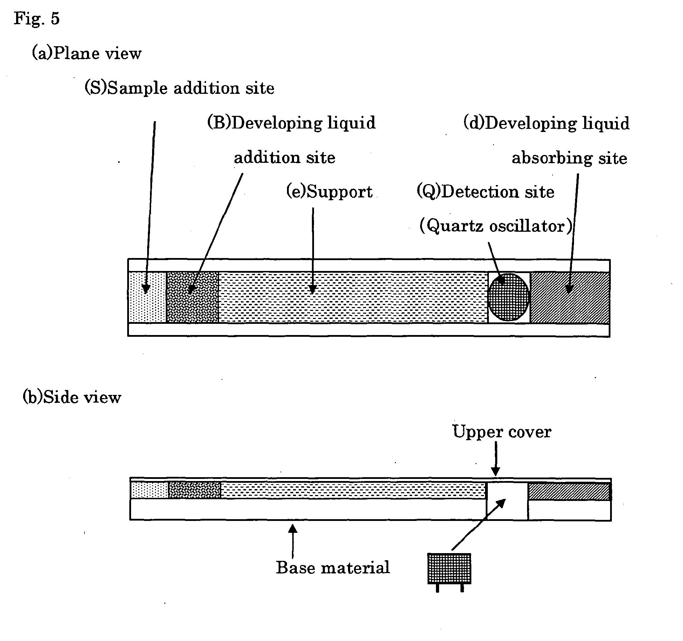

Fig. 5 is a schematic view illustrating one of specific embodiments of the device for measuring an object to be measured of the present invention: Fig. 5(a) shows the plane view; and Fig. 5(b) shows the side view.

This device comprises a sample addition site (S), a developing liquid addition site (B), a support (e), a detection site (Q) having a quartz oscillator sandwiched between two electrodes, and a developing liquid absorbing site (d) which are provided on a base material; the sample addition site, the developing liquid addition site, the support, the detection site and the developing liquid absorbing site being covered by an upper cover and connected so as to be movable by the capillary flow of the developing liquid. For the sample addition site and the developing liquid addition site may be used a material that is the same as the support, but different one may be also used. At the sample addition site, the upper cover has a pore provided such that the sample can be added thereto. At the developing liquid addition site, a pore may or may not be provided. Also, at the developing liquid addition site can be provided a binder immobilizing site where the binder is immobilized on the support so as not to be moved by the capillary flow of the developing liquid, a binder retaining site where the binder is retained so as to be movable by the capillary flow of the developing liquid, an analogue of the object to be measured immobilizing site where the analogue of the object to be measured is immobilized on the support so as not to be moved by the capillary flow of the developing liquid, or an analogue of the object to be measured retaining site where the analogue of the object to be measured is retained so as to be movable by the capillary flow of the developing liquid. The material for the base material is not particularly limited, but a resin is preferably used. For the developing liquid absorbing site can be used a water absorptive high molecular compound. Examples of the water absorptive high molecular compound include, cellulose, glassfiber, cotton, polyurethane and the like. A quartz oscillator insertion space is provided such that the quartz oscillator inserted in the detection site may be detachable.

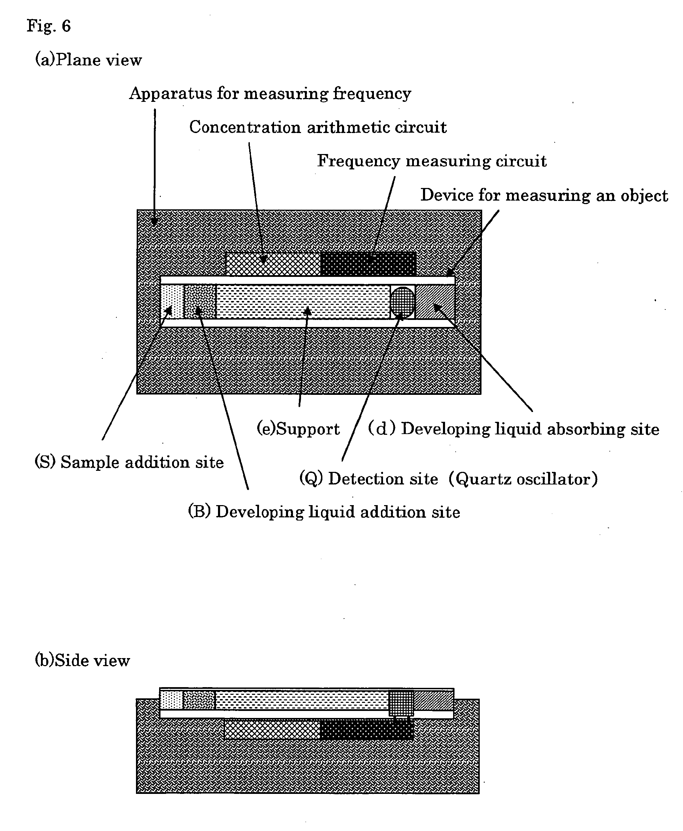

Fig. 6 is a schematic view illustrating one of specific embodiments of the apparatus for measuring an object to be measured of the present invention: Fig. 6(a) shows the plane view; and Fig. 6(b) shows the side view.

This apparatus comprises a device comprising a sample addition site (S), a developing liquid addition site (B), a support (e), a detection site (Q) having a quartz oscillator sandwiched between two electrodes, and a developing liquid absorbing site (d) which are provided on a base material; the sample addition site, the developing liquid addition site, the support, the detection site and the developing liquid absorbing site being covered by an upper cover and connected so as to be movable by the capillary flow of the developing liquid, and the device being connected to an apparatus for measuring frequency that has a frequency measuring circuit and a concentration arithmetic circuit via the electrode of the quartz oscillator of the device.

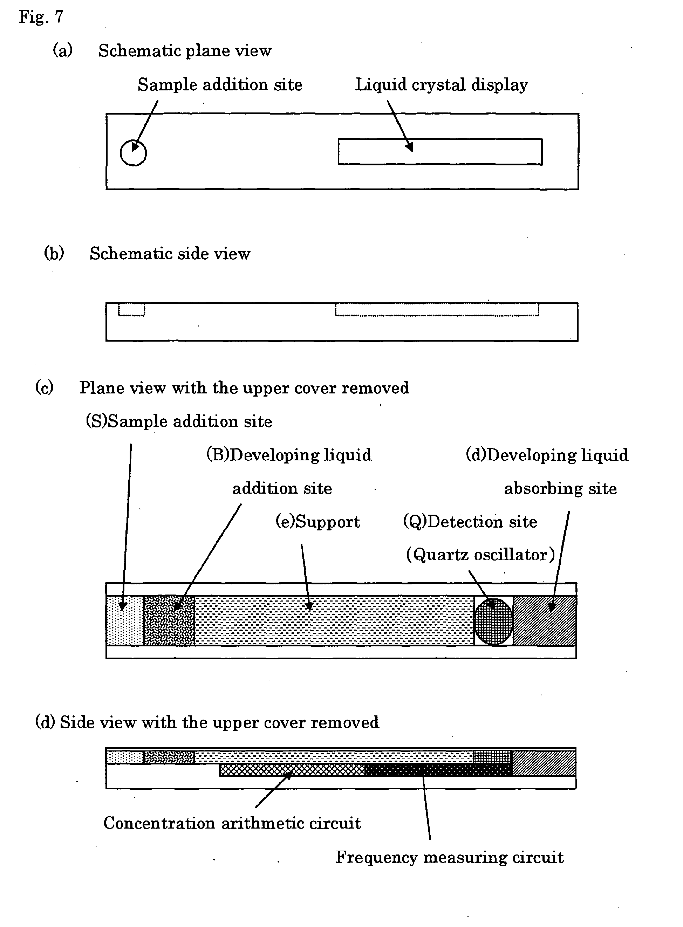

Fig. 7 is a schematic view illustrating one of specific embodiments of the apparatus for measuring an object to be measured of the present invention: Fig. 7(a) shows the schematic plane view; Fig. 7(b) shows the schematic side view; Fig. 7(c) shows the plane view with the upper cover removed; and Fig. 7(d) shows the side view with the upper cover removed.

This apparatus comprises a device comprising a sample addition site (S), a developing liquid addition site (B), a support (e), a detection site (Q) having a quartz oscillator sandwiched between two electrodes, and a developing liquid absorbing site (d) which are provided on a base material; the sample addition site, the developing liquid addition site, the support, the detection site and the developing liquid absorbing site being covered by an upper cover and connected so as to be movable by the capillary flow of the developing liquid, and in the base material of the device being provided an apparatus for measuring frequency that has a frequency measuring circuit and a concentration arithmetic circuit, with the device being connected to the apparatus via the electrode of the quartz oscillator.

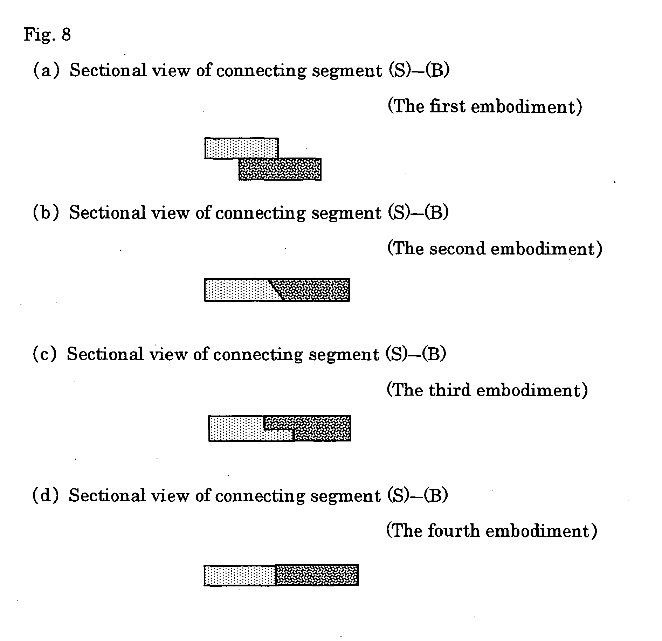

Fig. 8 schematically illustrates a connecting segment of the sample addition site (S) and the developing liquid addition site (B). Each member is constituted from a material that allows the developing liquid to be movable by the capillary flow and that may be the same or different, and can be disposed as diagrammatically shown in, for example, the first embodiment, the second embodiment, the third embodiment and the fourth embodiment.

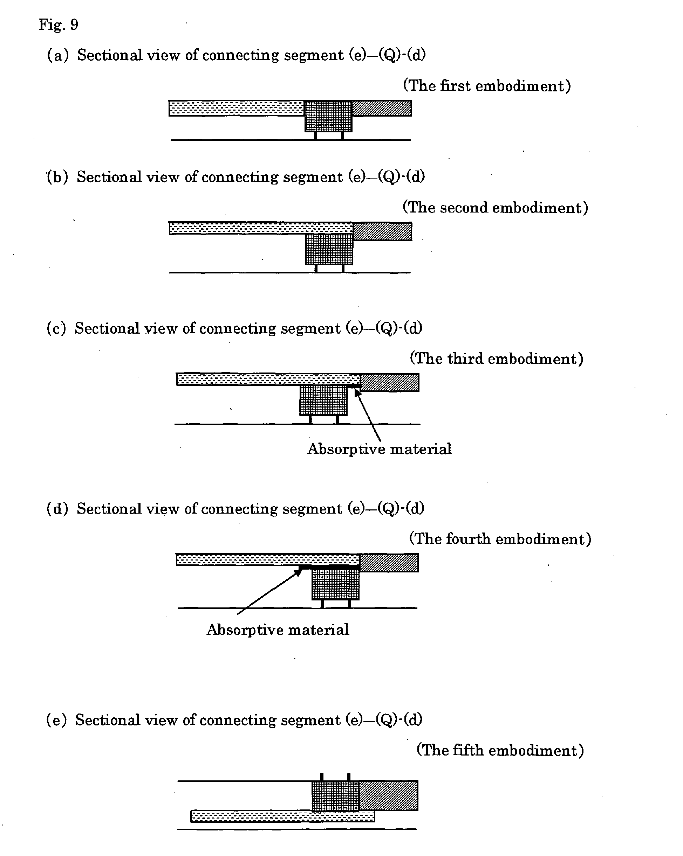

Fig. 9 schematically illustrates a connecting segment of the support (e), the detection site (Q) and the developing liquid absorbing site (d). Into the detection site is inserted the piezoelectric vibrator, and a fine gap between the upper cover, the support or the absorptive material and the piezoelectric vibrator allows the developing liquid to be movable from the support to the developing liquid absorbing site by the capillary flow. The absorptive material may be the same as the material constituting the developing liquid absorbing site.

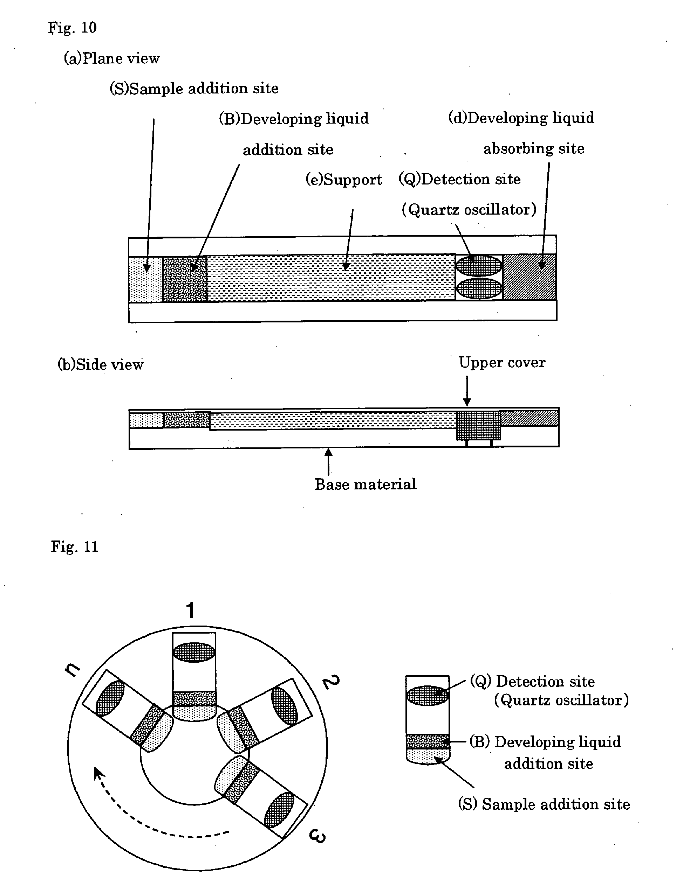

Fig. 10 is a schematic plane view illustrating one example of specific embodiments of the device for measuring an object to be measured of the present invention.

This device comprises a sample addition site (S), a developing liquid addition site (B), a support (e), a detection site (Q) having a quartz oscillator sandwiched between two electrodes, and a developing liquid absorbing site (d) which are provided on a base material; the sample addition site, the developing liquid addition site, the support, the detection site and the developing liquid absorbing site being covered by an upper cover and connected so as to be movable by the capillary flow of the developing liquid. In this device, two quartz oscillators are inserted at the detection site into the quartz oscillator insertion space.

In this example, two quartz oscillators are placed parallel with the direction of movement of the developing liquid, however, the two quartz oscillators can be also placed perpendicularly to the direction of movement of the developing liquid.

On one quartz oscillator is immobilized a trapper A (c1), an analogue of the object to be measured (c1'), a binder (c2) or a trapper B (c3), while none of the trapper A (c1), the analogue of the object to be measured (c1'), the binder (c2) and the trapper B (c3) are immobilized on another piezoelectric vibrator. Use of this apparatus enables correction with respect to the nonspecific reaction.

Fig. 11 illustrates an example of a device including radially disposed multiple devices for measuring the object to be measured, from 1 to n. By concomitantly adding a sample and a standard substance having a known concentration to the sample addition site, the calibration curve can be produced, and at the same time, the concentration of the substance to be measured in the sample can be determined.

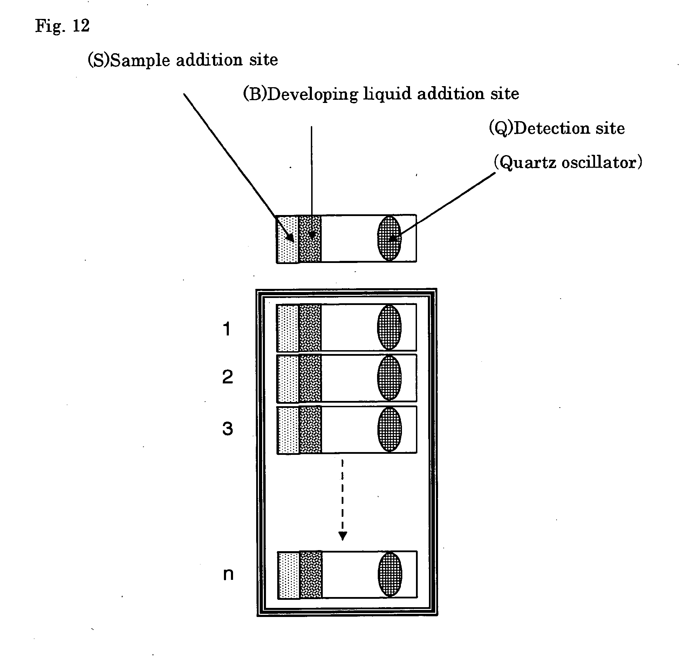

Fig. 12 illustrates an example of a device including multiple devices for measuring the object to be measured disposed in a rectangle, from 1 to n. By concomitantly adding a sample and a standard substance having a known concentration to the sample addition site, the calibration curve can be produced, and at the same time, the concentration of the substance to be measured in the sample can be determined.

The device for measuring an object to be measured in a sample of the present invention may be as in the following embodiments.

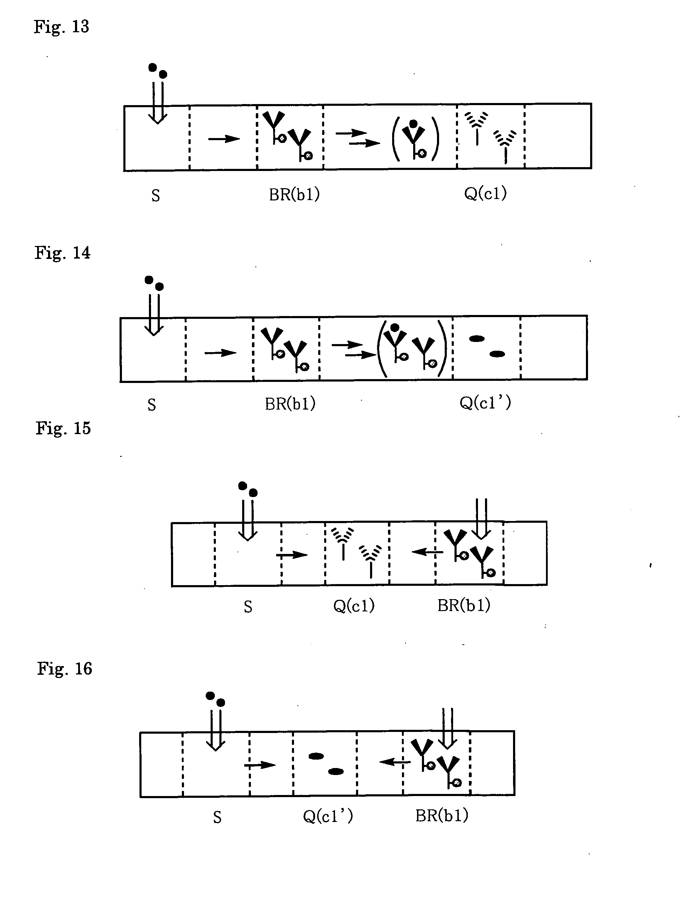

As the first example of the device for measuring an object to be measured (see, Fig. 13 and Fig. 14), for example, a device for measuring an object to be measured in a sample can be exemplified which has a sample addition site (S) provided on a support to allow the sample containing the object to be measured to be movable by the capillary flow of a developing liquid, a labeled binder (b1) in which a label is bound to a binder that binds to the object to be measured and which is retained on the support so as to be movable by the capillary flow of the developing liquid, and a piezoelectric vibrator on which a trapper A (c1) is immobilized that binds to the object to be measured or a piezoelectric vibrator on which an analogue of the object to be measured (c1') is immobilized, the device being designed such that the sample and the labeled binder move on the support by the capillary flow in the same direction toward the trapper A or the analogue of the object to be measured immobilized on the piezoelectric vibrator.

It is preferred that the labeled binder be retained previously at the binder retaining site (BR) on the support, however, a mode in which the labeled binder is supplied in a kit without being retained on the support may be acceptable. Additionally, a developing liquid addition site can be also provided between the sample addition site and the detection site in the device. The labeled binder can be used by adding from the sample addition site after dissolving in the developing liquid if necessary, together with or separately from the sample, or from the developing liquid addition site provided between the sample addition site and the detection part after dissolving in the developing liquid if necessary, at the same time with or different time from the timing of addition of the sample.

When the trapper A (c1) that binds to the object to be measured is immobilized on the piezoelectric vibrator (see, Fig. 13), the device may be used for methods of the measurement based on principles of so called sandwich measurement method, while when the analogue of the object to be measured (c1') that binds to the labeled binder is immobilized on the piezoelectric vibrator (see, Fig. 14), the apparatus may be used for methods of the measurement based on principles of so called competitive measurement method. Moreover, unlabeled binder may be also used in stead of the labeled binder.

As the second example of the device for measuring an object to be measured (see, Fig. 15 and Fig. 16), for example, a device for measuring an object to be measured in a sample can be exemplified which has a sample addition site (S) provided on a support to allow the sample containing the object to be measured to be movable by the capillary flow of a developing liquid, a labeled binder (b1) in which a label is bound to a binder that binds to the object to be measured and which is retained on the support so as to be movable by the capillary flow of the developing liquid, and a piezoelectric vibrator on which a trapper A (c1) is immobilized that binds to the object to be measured or a piezoelectric vibrator on which an analogue of the object to be measured (c1') is immobilized, the device being designed such that the sample and the labeled binder move on the support by the capillary flow in the reverse direction toward the trapper A or the analogue of the object to be measured immobilized on the piezoelectric vibrator.

It is preferred that the labeled binder be retained previously at the binder retaining site (BR) on the opposite side of the sample addition site with respect to the detection site on the support, however, a mode in which the labeled binder is supplied in a kit without being retained on the support may be acceptable. Additionally, a developing liquid addition site can be also provided on the opposite side of the sample addition site with respect to the detection site in the device. When the labeled binder has been previously retained on the support, the developing liquid addition site may or may not be provided, but to provide the developing liquid addition site is preferred. In this instance, the developing liquid is added from the developing liquid addition site, and the labeled binder can be allowed to move toward the detection site. When the labeled binder has not been previously retained on the support, the developing liquid addition site may be provided, and the labeled binder can be used by adding from the developing liquid addition site at the same time with or different time from the timing of addition of the sample.

When the trapper A (c1) that binds to the object to be measured is immobilized on the piezoelectric vibrator (see, Fig. 15), the device may be used for methods of the measurement based on principles of so called sandwich measurement method, while when the analogue of the object to be measured (c1') that binds to the labeled binder is immobilized on the piezoelectric vibrator (see, Fig. 16), the device may be used for methods of the measurement based on principles of so called competitive measurement method. Moreover, unlabeled binder may be also used in stead of the labeled binder.

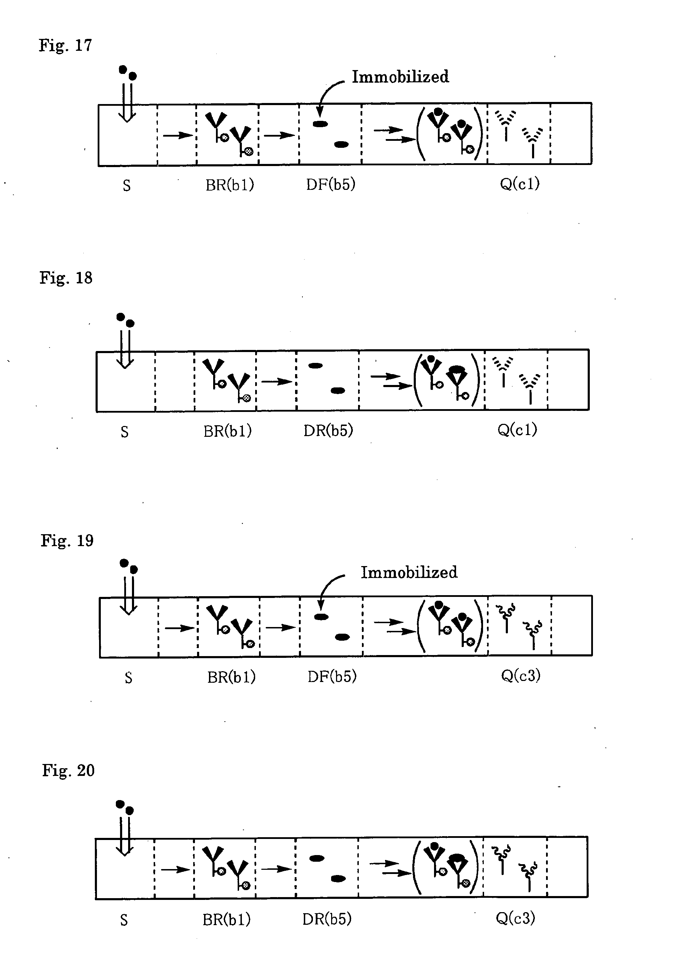

As the third example of the device for measuring an object to be measured (see, Fig. 17 or Fig. 18), for example, a device for measuring an object to be measured in a sample can be exemplified which has a sample addition site (S) provided on a support to allow the sample containing the object to be measured to be movable by the capillary flow, a labeled binder (b1) in which a label is bound to a binder that binds to the object to be measured and which is retained on the support so as to be movable by the capillary flow, an analogue of the object to be measured (b5) that binds to the labeled binder and is immobilized on the support or retained on the support so as to be movable, and a piezoelectric vibrator on which a trapper A (c1) that binds to the object to be measured bound to the labeled binder is immobilized, the device being designed such that the sample and the labeled binder are movable on the support by the capillary flow in the same direction toward the trapper A immobilized on the piezoelectric vibrator, and the analogue of the object to be measured being retained or immobilized between the sample addition site and the detection site on the support.

It is preferred that the analogue of the object to be measured is immobilized between the sample addition site and the detection site on the support, and the labeled binder is retained previously on the support, however, a mode in which the labeled binder is supplied in a kit without being retained on the support may be acceptable. Additionally, a developing liquid addition site can be also provided between the sample addition site and the immobilizing site of the analogue of the object to be measured in the device. The labeled binder may be used by adding from the sample addition site after dissolving in the developing liquid if necessary, together with or separately from the sample, or from the developing liquid addition site provided between the sample addition site and the binder immobilizing site after dissolving in the developing liquid if necessary, at the same time with or different time from the timing of addition of the sample.

Moreover, unlabeled binder may be also used in stead of the labeled binder. In this instance, a combination which does not cause a specific binding reaction between the analogue of the object to be measured (b5) and the trapper A (c1) is preferred.

As the fourth example of the device for measuring an object to be measured (see, Fig. 19 or Fig. 20), for example, a device for measuring an object to be measured in a sample can be exemplified which has a sample addition site (S) provided on a support to allow the sample containing the object to be measured to be movable by the capillary flow, a labeled binder (b1) in which a label is bound to a binder that binds to the object to be measured and which is retained on the support so as to be movable by the capillary flow, an analogue of the object to be measured (b5) that binds to the labeled binder and is immobilized on the support or retained on the support so as to be movable, and a piezoelectric vibrator on which a trapper B (c3) is immobilized that binds to the labeled binder, the device being designed such that the sample and the labeled binder are movable on the support by the capillary flow in the same direction toward the trapper B immobilized on the piezoelectric vibrator, and the analogue of the object to be measured being retained or immobilized between the sample addition site and the detection site on the support.

It is preferred that the analogue of the object to be measured is immobilized between the sample addition site and the detection site on the support, and the labeled binder is retained previously on the support, however, a mode in which the labeled binder is supplied in a kit without being retained on the support may be acceptable. Additionally, a developing liquid addition site can be also provided between the sample addition site and the retaining or immobilizing site of the analogue of the object to be measured in the device. The labeled binder may be used by adding from the sample addition site after dissolving in the developing liquid if necessary, together with or separately from the sample, or from the developing liquid addition site provided between the sample addition site and the binder immobilizing site after dissolving in the developing liquid if necessary, at the same time with or different time from the timing of addition of the sample.

Moreover, unlabeled binder may be also used in stead of the labeled binder. In this instance, a combination in which the analogue of the object to be measured (b5) and the object to be measured in the sample shall be different substances is preferred.

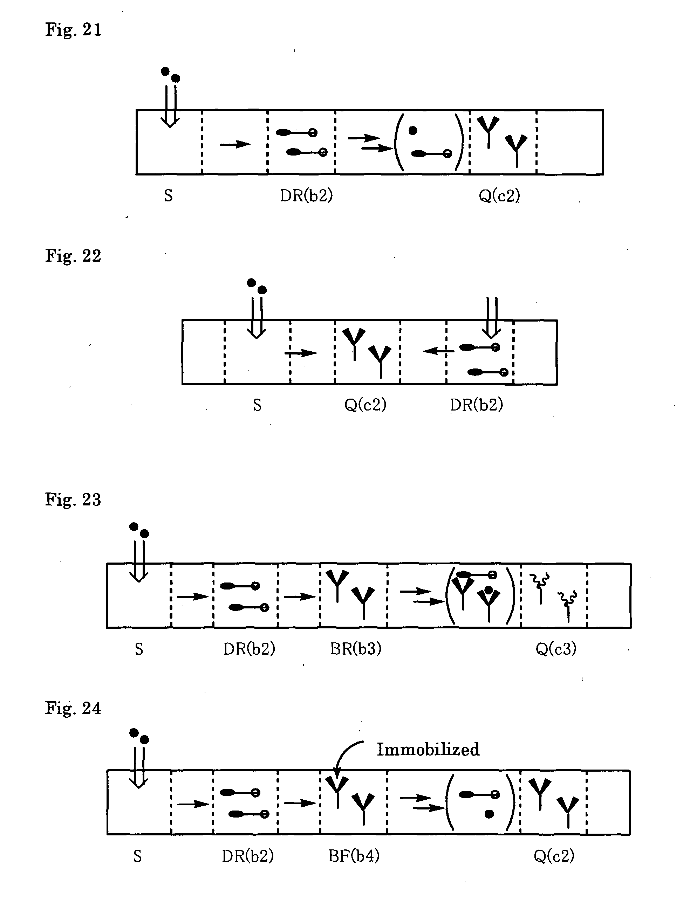

As the fifth example of the device for measuring an object to be measured (see, Fig. 21), for example, a device for measuring an object to be measured in a sample can be exemplified which includes a sample addition site (S) provided on a support to allow the sample containing the object to be measured to be movable by the capillary flow, a labeled analogue of the object to be measured (b2) in which a label is bound to an analogue of the object to be measured and which is retained on the support so as to be movable by the capillary flow, and a piezoelectric vibrator on which a binder (c2) that binds to the object to be measured and the labeled analogue of the object to be measured is immobilized, the device being designed such that the sample and the labeled analogue of the object to be measured move on the support by the capillary flow in the same direction toward the binder immobilized on the piezoelectric vibrator.

It is preferred that the labeled analogue of the object to be measured is retained previously on the support, however, a mode in which the labeled analogue of the object to be measured is supplied in a kit without being retained on the support may be acceptable. Additionally, a developing liquid addition site can be also provided between the sample addition site and the detection site in the device. The labeled analogue of the object to be measured may be used by adding from the sample addition site after dissolving in the developing liquid if necessary, together with or separately from the sample, or from the developing liquid addition site provided between the sample addition site and the detection part after dissolving in the developing liquid if necessary, at the same time with or different time from the timing of addition of the sample.

As the sixth example of the device for measuring an object to be measured (Fig. 22), for example, a device for measuring an object to be measured in a sample can be exemplified which includes a sample addition site (S) provided on a support to allow the sample containing the object to be measured to be movable by the capillary flow, a labeled analogue of the object to be measured (b2) in which a label is bound to an analogue of the object to be measured and which is retained on the support so as to be movable by the capillary flow, and a piezoelectric vibrator on which a binder (c2) that binds to the object to be measured and the labeled analogue of the object to be measured is immobilized, the device being designed such that the sample and the labeled analogue of the object to be measured move on the support by the capillary flow in the reverse direction toward the binder on the piezoelectric vibrator.

It is preferred that the labeled analogue of the object to be measured be retained previously on the opposite side of the sample addition site with respect to the detection site on the support, however, a mode of a kit in which the labeled analogue of the object to be measured is separated apart without being retained on the support may be acceptable. Additionally, a developing liquid addition site can be also provided on the opposite side of the sample addition site with respect to the detection site in the device. When the labeled analogue of the object to be measured has been previously retained on the support, the developing liquid addition site may or may not be provided, but to provide the developing liquid addition site is preferred. In this instance, the developing liquid is added from the developing liquid addition site, and the labeled analogue of the object to be measured is allowed to move toward the detection site. When the labeled analogue of the object to be measured has not been previously retained on the support, the developing liquid addition site may be provided, and the labeled analogue of the object to be measured may be used by adding from the developing liquid addition site at the same time with or different time from the timing of addition of the sample.

Moreover, unlabeled binder may be also used in stead of the labeled binder.

As the seventh example of the device for measuring an object to be measured (Fig. 23), for example, an apparatus for measuring an object to be measured in a sample can be exemplified which includes a sample addition site (S) provided on a support to allow the sample containing the object to be measured to be movable by the capillary flow, a labeled analogue of the object to be measured (b2) in which a label is bound to an analogue of the object to be measured and which is retained on the support so as to be movable by the capillary flow, a binder (b3) that binds to the object to be measured and the analogue of the object to be measured and is retained on the support so as to be movable by the capillary flow, and a piezoelectric vibrator on which a trapper B (c3) that binds to the binder bound to the labeled analogue of the object to be measured and the binder bound to the object to be measured, the device being designed such that the sample, the analogue of the object to be measured and the binder are movable on the support by the identical capillary flow toward the trapper B immobilized on the piezoelectric vibrator.

It is preferred that the labeled analogue of the object to be measured and the binder are retained previously on the support, however, a mode in which one or two selected from the group consisting of the labeled analogue of the object to be measured and the binder are not retained on the support but the component not retained on the support and the device are supplied in a kit may be acceptable. Additionally, a developing liquid addition site can be also provided between the sample addition site and the detection site in the device. The labeled analogue of the object to be measured and the binder may be used by adding from the sample addition site after dissolving in the developing liquid if necessary, together with the sample or each separately, or from the developing liquid addition site provided between the sample addition site and the detection site after dissolving in the developing liquid if necessary, at the same time with or different time from the timing of addition of the sample, respectively.

The site where the labeled analogue of the object to be measured is retained and the site where the binder is retained may be positioned between the sample addition site and the detection site, and they may be the identical site or different sites.

As the eighth example of the device for measuring an object to be measured (Fig. 24), for example, a device for measuring an object to be measured in a sample can be exemplified which includes a sample addition site (S) provided on a support to allow the sample containing the object to be measured to be movable by the capillary flow, a labeled analogue of the object to be measured (b2) in which a label is bound to an analogue of the object to be measured and which is retained on the support so as to be movable by the capillary flow, a binder (b4) that binds to the object to be measured and the analogue of the object to be measured and is immobilized on the support, and a piezoelectric vibrator on which a binder (c2) that binds to the object to be measured and the analogue of the object to be measured, the device being designed such that the sample and the analogue of the object to be measured are movable on the support by the capillary flow in the same direction toward the binder immobilized on the piezoelectric vibrator, and the binder being immobilized between the sample addition site and the detection site on the support.

It is preferred that the binder is immobilized between the sample addition site and the detection site on the support, and the labeled analogue of the object to be measured is retained previously on the support, however, a mode in which the labeled analogue of the object to be measured is not retained on the support but is supplied in a kit may be acceptable. Additionally, a developing liquid addition site can be also provided between the sample addition site and the binder immobilizing site in the device. The labeled analogue of the object to be measured may be used by adding from the sample addition site after dissolving in the developing liquid if necessary, together with the sample or separately, or from the developing liquid addition site provided between the sample addition site and the binder immobilizing site after dissolving in the developing liquid if necessary, at the same time with or different time from the timing of addition of the sample.

Furthermore, in each of the devices for measuring the object to be measured according to the first to eighth embodiments described above, two piezoelectric vibrators may be disposed in combination at the detection site or the developing liquid addition site may be provided as illustrated in Fig. 10; or a control line may be provided as illustrated in Fig. 4.

The device of the present invention having two piezoelectric vibrators at the detection site falls under a particularly preferred embodiment, and in this instance, it is characterized in that any one of the trapper A (c1), the analogue of the object to be measured (c1'), the binder (c2) or the trapper B (c3) is immobilized on one piezoelectric vibrator, while none of them is immobilized on another piezoelectric vibrator. Use of this device enables reduction of inaccuracy of measurement derived from the nonspecific reaction caused due to the sample containing the object to be measured, thereby permitting more accurate measurement which is not affected by any nonspecific reaction.

In addition, the control line is a site for verifying occurrence of the intended reaction, and can be provided at an arbitrary position on the support. The control line can be formed by, for example, immobilizing the trapper B at the developing liquid absorbing site or an arbitrary position on the support.

According to the aforementioned examples of the device for measuring the object to be measured shown in Figs. 13 to 24, as described above, an apparatus can be constructed by connecting the frequency measuring circuit or the frequency measuring circuit in which the frequency measuring circuit and the concentration arithmetic apparatus are connected.

Size of the device of the present invention is not particularly limited, but has preferably a width of 0.01 to 30 cm, a length of 0.1 to 30 cm and a thickness of 1 to 10,000 µm, more preferably a width of 0.1 to 10 cm, a length of 0.5 to 15 cm and a thickness of 10 to 1,000 µm, and particularly preferably a width of 0.2 to 5 cm, a length of 1.0 to 10 cm, and a thickness of 50 to 500 µm.

As the method for measuring an object to be measured in a sample of the present invention, for example, the methods of the following embodiments may be exemplified.

As the first example of the method of the measurement (see, Fig. 13 and Fig. 14), for example, a method for measuring an object to be measured in a sample can be exemplified which comprises: (1) supplying the object to be measured in a sample and a labeled binder (b1) that binds to the object to be measured by the capillary flow in the same direction to a trapper A (c1) or an analogue of the object to be measured (c1') immobilized on a piezoelectric vibrator; (2) allowing the labeled binder and the object to be measured to react with the trapper A or the analogue of the object to be measured to form a reaction complex; (3) measuring frequency of the piezoelectric vibrator at an arbitrary time point after initiating the reaction; and (4) determining the concentration of the object to be measured in the sample from the frequency measured in the step (3) and a calibration curve showing a relationship between the frequency and the concentration produced using the object to be measured having a known concentration.

As this method of the measurement, for example, a method in which the first embodiment of the device for the measurement described above is used may be exemplified.

In this method of the measurement, the object to be measured in the sample may be moved on the support by the capillary flow together with the labeled binder, but the object to be measured in the sample and the labeled binder may be also allowed to move with a time lag. As the process for allowing the object to be measured in the sample and the labeled binder to move together on the support by the capillary flow, for example, a process in which the sample is added on the support where the labeled binder has been already retained upon addition of the sample, a process in which a mixture obtained by mixing the sample and the labeled binder to the sample addition site, or the like may be exemplified. As the process for allowing the object to be measured in the sample and the labeled binder to move with a time lag, for example, a process in which the sample is added to the sample addition site, followed by adding the labeled binder to the same site, or the like may be exemplified.

When the piezoelectric vibrator on which the trapper A was immobilized is used, a complex which contains the trapper A, the object to be measured and the labeled binder is formed on the piezoelectric vibrator. To the contrary, when the piezoelectric vibrator on which the analogue of the object to be measured was immobilized is used, a complex which contains the analogue of the object to be measured and the labeled binder is formed on the piezoelectric vibrator.

The frequency of the piezoelectric vibrator on which the complex was immobilized is measured, while the frequency of the piezoelectric vibrator before the object to be measured in the sample is moved together with the labeled binder on the support by the capillary flow is measured, and then difference in the two frequencies thus measured (blank subtracted frequency) is calculated.

Concentration of the object to be measured in the sample can be measured from this blank subtracted frequency and a calibration curve produced using a solution of the object to be measured having a known concentration (calibration curve showing a relationship between the concentration of the object to be measured and the blank subtracted frequency).

As the second example of the method of the measurement (see, Fig. 15 and Fig. 16), for example, a method for measuring an object to be measured can be exemplified which comprises: (1) allowing the object to be measured and a labeled binder to move on a support by the capillary flow in the mutually reverse direction toward a piezoelectric vibrator on which a trapper A that binds to the object to be measured bound to the labeled binder was immobilized, or a piezoelectric vibrator on which an analogue of the object to be measured that binds to the labeled binder was immobilized; (2) allowing a sandwich reaction or a competitive reaction to proceed on the piezoelectric vibrator on which the trapper A that binds to the object to be measured bound to the labeled binder was immobilized, or the piezoelectric vibrator on which the analogue of the object to be measured that binds to the labeled binder was immobilized; (3) measuring frequency of the piezoelectric vibrator following the step (2); and (4) measuring the concentration of the object to be measured in the sample from the frequency measured in the step (3) and a calibration curve produced using the object to be measured having a known concentration.

As this method of the measurement, for example, a method in which the second embodiment of the device for the measurement described above is used may be exemplified.

In this method of the measurement, the object to be measured in the sample moves in a direction the reverse of the movement of the labeled binder on the support. Any condition that permits the perfect to be measured in the sample and the labeled binder to undergo the sandwich reaction or the competitive reaction on the piezoelectric vibrator is preferred. Such conditions may involve, for example, an embodiment in which addition of the sample to the sample addition site and addition of the developing liquid to the developing liquid addition site are concomitantly conducted when the labeled binder has been previously retained on the support, and an embodiment in which addition of the sample to the sample addition site and addition of a solution containing the labeled binder to the developing liquid addition site are concomitantly conducted when the labeled binder had not been previously retained on the support.

When the piezoelectric vibrator on which the trapper A was immobilized is used, a complex which contains the trapper A, the object to be measured and the labeled binder is formed on the piezoelectric vibrator. To the contrary, when the piezoelectric vibrator on which the analogue of the object to be measured was immobilized is used, a complex which contains the analogue of the object to be measured and the labeled binder is formed on the piezoelectric vibrator.

The frequency of the piezoelectric vibrator on which the complex was immobilized is measured. On the other hand, the frequency of the piezoelectric vibrator before the object to be measured in the sample is moved together with the labeled binder on the support by the capillary flow is measured, and then difference in the two frequencies thus measured (blank subtracted frequency) is calculated.

Concentration of the object to be measured in the sample may be measured from the difference in this blank subtracted frequency and a calibration curve produced using a solution of the object to be measured having a known concentration (calibration curve showing a relationship between the concentration of the object to be measured and the blank subtracted frequency).

As the third example of the method of the measurement (see, Fig. 17 and Fig. 18), for example, a method for measuring an object to be measured in a sample can be exemplified which comprises: (1) allowing on a support a competitive reaction of the object to be measured and an analogue of the object to be measured (b5) retained or immobilized on the support against to a labeled binder (b1); (2) allowing a component not captured by the analogue of the object to be measured in the competitive reaction, or a component captured by the analogue of the object to be measured retained so as to be movable on the support in the step (1) to move by the capillary flow on the support, and further to pass by a piezoelectric vibrator on which a trapper A (c1) that binds to the object to be measured and the analogue of the object to be measured was immobilized; (3) measuring frequency of the piezoelectric vibrator following the step (2); and (4) measuring the concentration of the object to be measured in the sample from the frequency measured in the step (3) and a calibration curve produced using the object to be measured having a known concentration.

As this method of the measurement, for example, a method in which the third embodiment of the device for the measurement described above is used may be exemplified.

In this method of the measurement, a complex containing the labeled binder and the object to be measured not captured on the support in the competitive reaction on the support, or a complex containing the analogue of the object to be measured and the labeled binder is captured on the piezoelectric vibrator on which the trapper A was immobilized. Embodiment of the competitive reaction on the support may involve a process in which the sample is added to the sample addition site when the labeled binder has been previously retained on the support (retained in a region between the sample addition site and the site where the analogue of the object to be measured was retained) or the like, and a process in which a mixed solution of the sample and the labeled binder is added to the sample addition site when the labeled binder has not been previously retained on the support.

On the piezoelectric vibrator is immobilized a complex which contains the trapper A, the object to be measured and the labeled binder, or a complex which contains the trapper A, the analogue of the object to be measured and the labeled binder. The frequency of the piezoelectric vibrator on which the complex was immobilized is measured, while the frequency of the piezoelectric vibrator before occurrence of the competitive reaction on the support is measured, and then difference in the two frequencies thus measured (blank subtracted frequency) is calculated.

Concentration of the object to be measured in the sample may be measured from this blank subtracted frequency and a calibration curve produced using a solution of the object to be measured having a known concentration (calibration curve showing a relationship between the concentration of the object to be measured and the blank subtracted frequency). In this example, a combination in which the analogue of the object to be measured (b5) and the trapper A (c1) do not cause a specific binding reaction is preferred.

As the fourth example of the method of the measurement (see, Fig. 19 and Fig. 20), for example, a method for measuring an object to be measured in a sample can be exemplified which comprises: (1) allowing on a support a competitive reaction of the object to be measured and an analogue of the object to be measured (b5) against to a labeled binder (b1); (2) allowing a complex that contains the object to be measured and the labeled binder, or a complex that contains the analogue of the object to be measured and the labeled binder in the competitive reaction in the step (1) to move by the capillary flow on the support, and further to pass by a piezoelectric vibrator on which a trapper B (c3) that binds to the binder bound to the object to be measured was immobilized; (3) measuring frequency of the piezoelectric vibrator following the step (2); and (4) measuring the concentration of the object to be measured in the sample from the frequency measured in the step (3) and a calibration curve produced using the object to be measured having a known concentration.

As this method of the measurement, for example, a method in which the fourth embodiment of the device for the measurement described above is used may be exemplified.

In this method of the measurement, a complex containing the labeled binder and the object to be measured not captured on the support in the competitive reaction on the support is captured on the piezoelectric vibrator on which the trapper B was immobilized. Embodiment of the competitive reaction on the support may involve a process in which the sample is added to the sample addition site when the labeled binder has been previously retained on the support (retained in a region between the sample addition site and the site where the analogue of the object to be measured was immobilized) or the like, and a process in which a mixed solution of the sample and the labeled binder is added to the sample addition site when the labeled binder has not been previously retained on the support, or the like.

On the piezoelectric vibrator is immobilized a complex which contains the trapper B, the object to be measured and the labeled binder, or a complex which contains the trapper B, the analogue of the object to be measured and the labeled binder. The frequency of the piezoelectric vibrator on which the complex was immobilized is measured, while the frequency of the piezoelectric vibrator before occurrence of the competitive reaction on the support is measured, and then difference in the two frequencies thus measured (blank subtracted frequency) is calculated.

Concentration of the object to be measured in the sample may be measured from this blank subtracted frequency and a calibration curve produced using a solution of the object to be measured having a known concentration (calibration curve showing a relationship between the concentration of the object to be measured and the blank subtracted frequency). In this example, a combination in which the complex containing the analogue of the object to be measured (b5) and the labeled binder has a reactivity to the trapper B which is different from the reactivity of the complex containing the object to be measured and the labeled binder is preferred.

As the fifth example of the method of the measurement (see, Fig. 21), for example, a method for measuring an object to be measured in a sample can be exemplified which comprises: (1) allowing the object to be measured in the sample and a labeled analogue of the object to be measured (b2) to move on a support by the capillary flow; (2) allowing the moved solution to further pass by a piezoelectric vibrator on which a binder (c2) was immobilized; (3) measuring frequency of the piezoelectric vibrator following the step (2); and (4) measuring the concentration of the object to be measured in the sample from the frequency measured in the step (3) and a calibration curve produced using the object to be measured having a known concentration.

As this method of the measurement, for example, a method in which the fifth embodiment of the device for the measurement described above is used may be exemplified.

In this method of the measurement, any condition that permits the object to be measured in the sample and the labeled analogue of the object to be measured to perfect the competitive reaction on the piezoelectric vibrator is preferred. Such conditions may involve, for example, an embodiment in which the sample is added to the sample addition site when the labeled analogue of the object to be measured has been previously retained on the support, or an embodiment in which a mixture of the sample and the labeled analogue of the object to be measured is added to the sample addition site when the labeled analogue of the object to be measured has not been previously retained on the support.

On the piezoelectric vibrator are formed a complex containing the object to be measured and the binder, and a complex containing the labeled analogue of the object to be measured and the binder. The frequency of the piezoelectric vibrator on which these complexes were immobilized is measured. On the other hand, the frequency of the piezoelectric vibrator before the object to be measured in the sample is moved together with the labeled analogue of the object to be measured on the support by the capillary flow is measured, and then difference in the two frequencies thus measured (blank subtracted frequency) is calculated.

Herein, the frequency derived from the complex containing the object to be measured and the binder is extremely small compared to the frequency derived from the complex containing the labeled analogue of the object to be measured and the binder. The frequency of the piezoelectric vibrator before the object to be measured in the sample moves together with the labeled analogue of the object to be measured on the support by the capillary flow is constant. Therefore, also in connection with the blank subtracted frequency, the blank subtracted frequency derived from the complex containing the object to be measured and the binder is significantly smaller than the blank subtracted frequency derived from the complex containing the labeled analogue of the object to be measured and the binder. Therefore, the blank subtracted frequency derived from the piezoelectric vibrator on which the complex containing the object to be measured and the binder, and the complex containing the labeled analogue of the object to be measured and the binder were immobilized can be assumed to be almost equal to the blank subtracted frequency derived from the piezoelectric vibrator on which the complex containing the labeled analogue of the object to be measured and the binder was immobilized.

Concentration of the object to be measured in the sample may be measured from the difference in this blank subtracted frequency and a calibration curve produced using a solution of the object to be measured having a known concentration (calibration curve showing a relationship between the concentration of the object to be measured and the blank subtracted frequency).

As the sixth example of the method of the measurement (see, Fig. 22), for example, a method for measuring an object to be measured in a sample can be exemplified which comprises: (1) allowing the object to be measured and a labeled analogue of the object to be measured (b2) to move on a support by the capillary flow in the mutually reverse direction toward a piezoelectric vibrator on which a binder (c2) was immobilized that binds to the object to be measured and the analogue of the object to be measured (b2); (2) allowing a competitive reaction to proceed on the piezoelectric vibrator on which the binder was immobilized; (3) measuring frequency of the piezoelectric vibrator following the step (2); and (4) measuring the concentration of the object to be measured in the sample from the frequency measured in the step (3) and a calibration curve produced using the object to be measured having a known concentration.

As this method of the measurement, for example, a method in which the sixth embodiment of the device for the measurement described above is used may be exemplified.

In this method of the measurement, the object to be measured in the sample moves in a direction the reverse of the movement of the labeled analogue of the object to be measured on the support. Any condition that permits the object to be measured in the sample and the labeled analogue of the object to be measured to perfect the competitive reaction on the piezoelectric vibrator is preferred. Such conditions may involve, for example, an embodiment in which addition of the sample to the sample addition site and addition of the developing liquid to the developing liquid addition site are concomitantly conducted when the labeled analogue of the object to be measured has been previously retained on the support, and an embodiment in which addition of the sample to the sample addition site and addition of a solution containing the labeled analogue of the object to be measured to the developing liquid addition site are concomitantly conducted when the labeled analogue of the object to be measured has not been previously retained on the support.

On the piezoelectric vibrator are formed a complex containing the object to be measured and the binder, and a complex containing the labeled analogue of the object to be measured and the binder. As described in the above method of the measurement 3, the blank subtracted frequency derived from the piezoelectric vibrator on which the complex containing the object to be measured and the binder, and the complex containing the labeled analogue of the object to be measured and the binder were immobilized can be assumed to be almost equal to the blank subtracted frequency derived from the piezoelectric vibrator on which the complex containing the labeled analogue of the object to be measured and the binder was immobilized.

Concentration of the object to be measured in the sample may be measured from the difference in this blank subtracted frequency and a calibration curve produced using a solution of the object to be measured having a known concentration (calibration curve showing a relationship between the concentration of the object to be measured and the blank subtracted frequency).

As the seventh example of the method of the measurement (see, Fig. 23), for example, a method for measuring an object to be measured in a sample can be exemplified which comprises: (1) allowing on a support a competitive reaction of the object to be measured in the sample and a labeled analogue of the object to be measured (b2) with respect to a binder (b3); (2) allowing a reaction product formed in the competitive reaction in the step (1) to move by the capillary flow on the support, and further to pass by a piezoelectric vibrator on which a trapper B (c3) that binds to the binder bound to the object to be measured and/or the binder bound to the labeled analogue of the object to be measured was/were immobilized; (3) measuring frequency of the piezoelectric vibrator following the step (2); and (4) measuring the concentration of the object to be measured in a sample from the frequency measured in the step (3) and a calibration curve produced using the object to be measured having a known concentration.

As this method of the measurement, for example, a method in which the seventh embodiment of the device for the measurement described above is used may be exemplified.

In this method of the measurement, a complex containing the object to be measured and the binder, and a complex containing the labeled analogue of the object to be measured and the binder which were formed in the competitive reaction on the support move on the support by the capillary flow, and these complexes are captured by the trapper B on the piezoelectric vibrator. Embodiment of the competitive reaction on the support may involve a process in which the sample is added to the support at the sample addition site on the support when both of the labeled analogue of the object to be measured and the binder has been previously retained on the support, or the like, and a process in which a mixed solution of the sample and another component is added to the support at the sample addition site on the support when only either one of the labeled analogue of the object to be measured or the binder has been retained on the support, or the like. Moreover, a process in which a mixed solution of the sample, the labeled analogue of the object to be measured and the binder is added to the sample addition site on the support when both of the labeled analogue of the object to be measured and the binder has not been retained on the support, or the like may be exemplified.

On the piezoelectric vibrator are immobilized a complex containing the labeled analogue of the object to be measured, the binder and the trapper B, and a complex containing the object to be measured, the binder and the trapper B. Similarly to the case of the above method of the measurement 3, the blank subtracted frequency derived from the piezoelectric vibrator on which the complex containing the labeled analogue of the object to be measured, the binder and the trapper B, and the complex containing the object to be measured, the binder and the trapper B were immobilized can be assumed to be almost equal to the blank subtracted frequency derived from the piezoelectric vibrator on which the complex containing the labeled analogue of the object to be measured, the binder and the trapper B was immobilized.

Concentration of the object to be measured in the sample may be measured from the difference in this blank subtracted frequency and a calibration curve produced using a solution of the object to be measured having a known concentration (calibration curve showing a relationship between the concentration of the object to be measured and the blank subtracted frequency).

As the eighth example of the method of the measurement (see, Fig. 24), for example, a method for measuring an object to be measured in a sample can be exemplified which comprises: (1) allowing on a support a competitive reaction of the object to be measured and a labeled analogue of the object to be measured (b2) with respect to a binder (b4) immobilized on the support; (2) allowing a component not captured by the binder in the competitive reaction in the step (1) to move by the capillary flow on the support, and further to pass by a piezoelectric vibrator on which the binder was immobilized; (3) measuring frequency of the piezoelectric vibrator following the step (2); and (4) measuring the concentration of the object to be measured in a sample from the frequency measured in the step (3) and a calibration curve produced using the object to be measured having a known concentration.

As this method of the measurement, for example, a method in which the eighth embodiment of the device for the measurement described above is used may be exemplified.

In this method of the measurement, the object to be measured and the labeled analogue of the object to be measured which has not been captured on the support in the competitive reaction on the support are captured on the piezoelectric vibrator on which the binder was immobilized. Embodiment of the competitive reaction on the support may involve a process in which the sample is added to the sample addition site when the labeled analogue of the object to be measured has been previously retained on the support (retained in a region between the sample addition site and the site where the binder was immobilized), and a process in which a mixed solution of the sample and the labeled analogue of the object to be measured is added to the sample addition site when the labeled analogue of the object to be measured has not been previously retained on the support, or the like. On the piezoelectric vibrator are immobilized a complex containing the labeled analogue of the object to be measured and the binder, and a complex containing the object to be measured and the binder. As described in the above method of the measurement 3, the blank subtracted frequency derived from the piezoelectric vibrator on which the complex containing the labeled analogue of the object to be measured and the binder, and the complex containing the object to be measured and the binder were immobilized can be assumed to be almost equal to the blank subtracted frequency derived from the piezoelectric vibrator on which the complex containing the labeled analogue of the object to be measured and the binder and the trapper B was immobilized.

Concentration of the object to be measured in the sample may be measured from the difference in this blank subtracted frequency and a calibration curve produced using a solution of the object to be measured having a known concentration (calibration curve showing a relationship between the concentration of the object to be measured and the blank subtracted frequency).

Furthermore, in each of the methods of the measurement according to the first to eighth embodiments described above, the measurement may be also performed using an apparatus having two piezoelectric vibrators disposed in combination at the detection site, an apparatus having a developing liquid absorbing site provided as illustrated in Fig. 10, or an apparatus having a control line provided as illustrated in Fig. 4.

The method of the measurement using the apparatus having two piezoelectric vibrators at the detection site falls under a particularly preferred embodiment, and in this instance, any one of the trapper A (c1), the analogue of the object to be measured (c1') the binder (c2) or the trapper B (c3) is immobilized on the first piezoelectric vibrator, while a piezoelectric vibrator in which none of them is immobilized thereon is used as the second piezoelectric vibrator. After initiating the specific reaction by allowing the sample to the detection site according to, for example, any method described in the first to eighth method of the measurement (see, Fig. 13 to 24), frequency of the first piezoelectric vibrator and frequency of the second piezoelectric vibrator are measured, and the method is characterized in that the frequency of the second piezoelectric vibrator is used as a control. From the difference between the first frequency and the second frequency, the concentration of the substance to be measured can be determined based on the calibration curve produced from the data obtained by using the object to be measured having a previously known concentration. Use of this method enables reduction of inaccuracy of measurement derived from the nonspecific reaction caused due to the sample containing the object to be measured, thereby permitting more accurate measurement which is not affected by any nonspecific reaction.

In addition, the control line, for example, provided on the support as shown in Fig. 4, is a site for verifying occurrence of the intended reaction, and verification of the reaction at the control line can ascertain definite occurrence of the intended reaction. Occurrence of the reaction at the control line can be found by, for example, alteration in the color tone by means of the label.

According to the aforementioned first to eighth examples of the method of the measurement, use of the apparatus in which the frequency measuring circuit was connected or the frequency measuring circuit in which the frequency measuring circuit and the concentration arithmetic apparatus were connected was connected, as described above, enables determination of the frequency or the concentration.

Hereinafter, the present invention will be explained in more detail by way of Examples, however, these do not anyhow limit the scope of the present invention. In the following Examples, reagents and equipments of the manufacturers described below were employed:
anti-human CRP goat polyclonal antibody (IgG; manufactured by COSMO BIO Co., Ltd.), anti-human CRP mouse monoclonal antibody (IgG; manufactured by COSMO BIO Co., Ltd.), anti-human insulin mouse monoclonal antibody (IgG; manufactured by COSMO BIO Co., Ltd.), gold colloid-labeled anti-human immunoglobulin goat polyclonal antibody (IgG; manufactured by British Biocell International Co., Ltd.), CRP standard solution (manufactured by COSMO BIO Co., Ltd.), boric acid (manufactured by KANTO CHEMICAL CO., INC.), imidazole (manufactured by KANTO CHEMICAL CO., INC.), tris(hydroxymethyl)aminomethane (hereinafter, referred to as tris) (manufactured by KANTO CHEMICAL CO., INC.), 2-iminothiolane hydrochloride (manufactured by KANTO CHEMICAL CO., INC.), tween 20 (manufactured by KANTO CHEMICAL CO., INC.), BSA (manufactured by Oriental Yeast Co., Ltd.), polystyrene microsphere DS02B (manufactured by Bangs Laboratories, Inc.), glutaraldehyde (manufactured by KANTO CHEMICAL CO., INC.), hexane (manufactured by KANTO CHEMICAL CO., INC.), Sephadex G-25 column (manufactured by Amersham Bioscience K.K.), MicroDosa (manufactured by BioDot), BioJet Quanti 3000 (manufactured by BioDot), quartz oscillator sensor chip (27 MHz) (manufactured by Initium, Inc.), nitrocellulose membrane [AccuFlow G type (manufactured by Schleicher & Schuell); FF85 (manufactured by Schleicher & Schuell)], absorptive paper [300 type, 470 type (manufactured by Schleicher & Schuell)].

### Brief Description of the Drawings

Fig. 1 is a view illustrating one example of the device for measuring an object to be measured.
Fig. 2 is a view illustrating one example of the device for measuring an object to be measured.
Fig. 3 is a view illustrating one example of the device for measuring an object to be measured.
Fig. 4 is a view illustrating one example of the device for measuring an object to be measured.
Fig. 5 is a view illustrating one example of the device for measuring an object to be measured.
Fig. 6 is a view illustrating one example of the device for measuring an object to be measured.
Fig. 7 is a view illustrating one example of the device for measuring an object to be measured.
Fig. 8 is a view illustrating an example of connection between a sample addition site and a developing liquid addition site of the device for measuring an object to be measured.
Fig. 9 is a view illustrating an example of connection of a support, a detection site and a developing liquid absorbing site of the device for measuring an object to be measured.
Fig. 10 is a view illustrating one example of the device for measuring an object to be measured.
Fig. 11 is a view illustrating one example of the device for measuring an object to be measured.
Fig. 12 is a view illustrating one example of the device for measuring an object to be measured.
Fig. 13 is a schematic view illustrating a first example of the device for measuring an object to be measured incorporating a quartz oscillator on which a trapper A was immobilized.
Fig. 14 is a schematic view illustrating a first example of the device for measuring an object to be measured incorporating a quartz oscillator on which an analogue of the object to be measured was immobilized.
Fig. 15 is a schematic view illustrating a second example of the device for measuring an object to be measured incorporating a quartz oscillator on which a trapper A was immobilized.
Fig. 16 is a schematic view illustrating a second example of the device for measuring an object to be measured incorporating a quartz oscillator on which an analogue of the object to be measured was immobilized.
Fig. 17 is a schematic view illustrating a third example of the device for measuring an object to be measured incorporating a quartz oscillator on which a trapper A was immobilized.
Fig. 18 is a schematic view illustrating a third example of the device for measuring an object to be measured incorporating a quartz oscillator on which a trapper A was immobilized.
Fig. 19 is a schematic view illustrating a fourth example of the device for measuring an object to be measured incorporating a quartz oscillator on which a trapper B was immobilized.
Fig. 20 is a schematic view illustrating a fourth example of the device for measuring an object to be measured incorporating a quartz oscillator on which a trapper B was immobilized.
Fig. 21 is a schematic view illustrating a fifth example of the device for measuring an object to be measured incorporating a quartz oscillator on which a binder was immobilized.
Fig. 22 is a schematic view illustrating a sixth example of the device for measuring an object to be measured incorporating a quartz oscillator on which a binder was immobilized.
Fig. 23 is a schematic view illustrating a seventh example of the device for measuring an object to be measured incorporating a quartz oscillator on which a trapper B was immobilized.
Fig. 24 is a schematic view illustrating an eighth example of the device for measuring an object to be measured incorporating a quartz oscillator on which a binder was immobilized.
Fig. 25 is a view illustrating one example of the device for measuring an object to be measured manufactured in Example 1.
Fig. 26 is a calibration curve showing a relationship between CRP concentration in a sample and the amount of decrease in frequency in measurement of the CRP concentration in a sample using the device for measuring an object to be measured of Example 1.
Fig. 27 is a view illustrating a conventional device for immunochromatography used in Comparative Example 1.
Fig. 28 is a view illustrating one example of the device for measuring an object to be measured manufactured in Example 4.

### Description of Reference Signs

• Object to be measured
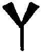 Binder
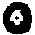 Label
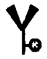 Labeled binder
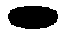 Analogue of the object to be measured
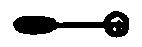 Labeled analogue of the object to be measured
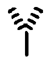 Trapper A
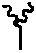 Trapper B

### Best Mode for Carrying Out the present invention

### Example 1: Device for measuring CRP

### (1) Immobilization of anti-human CRP goat polyclonal antibody (IgG) on quartz oscillator

An anti-human CRP goat polyclonal antibody (IgG) was dissolved in a 0.1 mol/L borate buffer (pH 8.0) to prepare a solution of 2.0 mg/mL of the anti-human CRP goat polyclonal antibody (IgG). To this solution (1.0 mL) was added a 1.0 mg/mL aqueous 2-iminothiolane solution (0.3 mL), and a reaction was allowed at 30°C for 0.5 hour. Thereafter, thus resulting reaction mixture was fractionated on column chromatography using Sephadex G-25 [elution solvent: 0.1 mmol/L borate buffer (pH 8.0)] to obtain a 0.2 mg/mL solution of the sulfhydrylated anti-human CRP goat polyclonal antibody (IgG) in the borate buffer.

This solution of the sulfhydrylated anti-human CRP goat polyclonal antibody (IgG) in borate buffer was added onto a surface of a quartz oscillator sensor chip (27 MHz) dropwise using MicroDosa BioJet Quanti 3000, and allowed to stand still at room temperature for 15 hours. Accordingly, the anti-human CRP goat polyclonal antibody (IgG) was immobilized on a gold electrode at the center of the quartz oscillator.

Furthermore, the gold electrode was washed with a 10 mmol /L phosphate buffer (pH 7.4) containing 0.1% tween 20 (0.1%) and 1% bovine serum albumin (hereinafter, referred to as BSA) to remove unreacted sulfhydrylated anti-human CRP goat polyclonal antibody (IgG).

### (2) Preparation of polystyrene microsphere-labeled anti-human CRP mouse monoclonal antibody (IgG)

To a 0.01 mg/mL anti-human CRP mouse monoclonal antibody (IgG) in phosphate-buffered physiological saline (10 mmol/L phosphate buffer, pH 7.2, containing 0.15 mol/L sodium chloride) (hereinafter, referred to as PBS) (1 mL) was added a 0.5% aqueous solution (10 mL) of polystyrene microsphere DS02B (manufactured by Bangs Laboratories, Inc.) having a particle size of 0.5 µm or less. After allowing to stand still at 37°C for 2 hours, the mixture was subjected to centrifugal separation at 4°C, 12000 rpm for 1 hour. To a precipitate obtained by removing the supernatant [precipitate containing polystyrene microsphere-labeled anti-human CRP mouse monoclonal antibody (IgG)] was added a 10 mmol/L imidazole buffer (50 mL), pH 7.8, containing 0.6% BSA, and dispersion of the mixture was allowed by an ultrasonic treatment to prepare a labeled solution.

### (3) Production of member for retaining labeled binder

After allowing impregnation of the labeled solution prepared in the paragraph (2) to a nitrocellulose membrane (AccuFlow G type) at room temperature for 1 hour, the membrane was vacuum dried at room temperature and 3 mmHg or less to produce a member for retaining labeled binder in which the polystyrene microsphere-labeled anti-human CRP mouse monoclonal antibody (IgG) was retained.

### (4) Production of device for measuring CRP

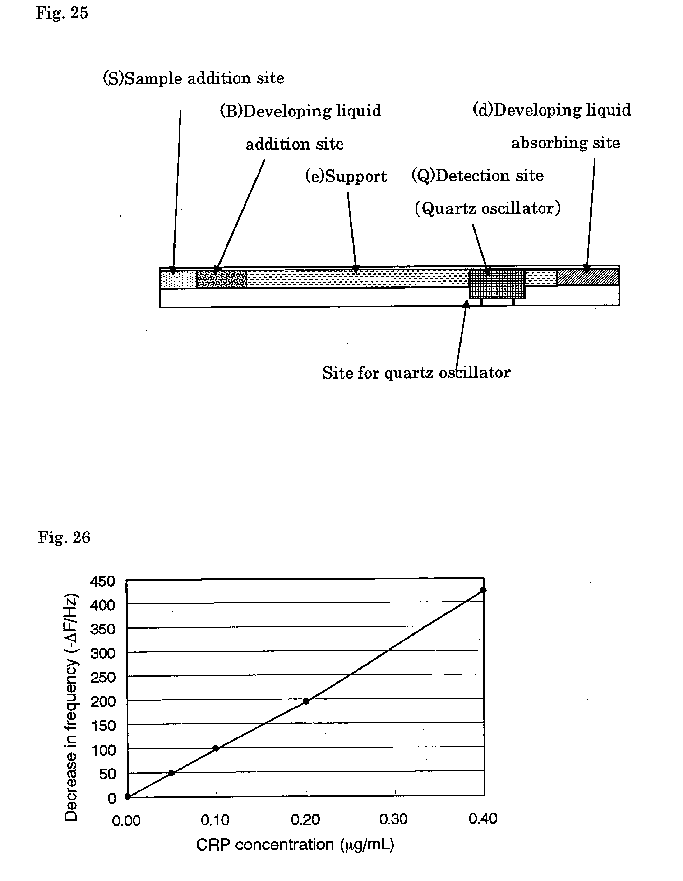

A member for sample addition which consists of cotton (manufactured by Schleicher & Schuell) and which is for adding a sample, the member for retaining labeled binder produced in the paragraph (3), a support consisting of a nitrocellulose membrane (FF85), a developing liquid absorbing member consisting of a quartz oscillator and absorptive paper 300 (manufactured by Schleicher & Schuell) on which the anti-human CRP goat polyclonal antibody (IgG) produced in the paragraph (1) was immobilized, were disposed at a sample addition site, a developing liquid addition site, a support, a detection site and a developing liquid absorbing site, respectively, to produce a device for measuring CRP as shown in Fig. 25. Example 2: Measurement of CRP concentration in sample using device for measuring CRP

### (1) Production of calibration curve

A sample (5 µL) was added dropwise to the sample addition site shown in Fig. 25. Next, a 10 mmol/L tris buffer, pH 7.5 was continuously added from the same site. The buffer reached to the retaining member to which the microsphere-labeled anti-human CRP mouse monoclonal antibody (IgG) was bound while diluting the sample, and at the retaining member, CRP in the sample reacts with the microsphere-labeled anti-human CRP mouse monoclonal antibody (IgG). The reaction liquid further proceeded the membrane by the capillary flow, and reached to a fine space on the quartz oscillator to fill in the space. On this quartz oscillator, a complex of CRP and the microsphere-labeled anti-human CRP mouse monoclonal antibody (IgG) reacts with the anti-human CRP goat polyclonal antibody (IgG) immobilized on the quartz oscillator. In proportion to the CRP concentration in the sample, the microsphere-labeled anti-human CRP mouse monoclonal antibody (IgG) is immobilized on the quartz oscillator. After completing absorption of the reaction liquid to the member for absorbing the reaction liquid, the quartz oscillator is removed, and attached to AFFINIX Q (manufactured by Initium, Inc.), a 10 mmol/L tris buffer, pH 7.5 was added to the vessel, and the frequency was measured 5 min later.

As the sample, <1> 10 mmol/L tris buffer, pH 7.5, alone, <2> 0.05 µg/mL CRP standard solution, <3> 0.1 µg/mL CRP standard solution, <4> 0.2 µg/mL CRP standard solution, and <5> 0.4 µg/mL CRP standard solution were used, respectively. For each sample, the quartz oscillator was replaced to measure the frequency. First, the frequency prior to adding the sample was measured, and then, thereto was added <1> 10 mmol/L tris buffer, pH 7.5 alone (5 µL). The frequency after absorption of the buffer to the absorptive pad was measured, and difference between both frequencies was calculated (this difference in frequency being defined as Δ1). Next, each sample (5 µL) of <2> to <5> was added in stead of <1> 10 mmol/L tris buffer, pH 7.5 (5 µL), and similarly to the case of <1>, the frequency prior to adding each sample, and the frequency after absorption of the reaction liquid to the absorptive pad were measured to calculate the difference in both frequencies (difference in frequency corresponding to each sample being defined as Δ2 to Δ5). Relationship between values obtained by subtracting Δ1 from each difference in frequency (Δ2 to Δ5), i.e., amount of decrease in frequency obtained by subtracting blank (-ΔF/Hz), and CRP concentration is shown in Table 1 and Fig. 26.

**Table 1**

| CRP concentration (µg/mL) | Amount of decrease in frequency (-ΔF/Hz) |
|---|---|
| 0.00 | 0 |
| 0.05 | 48 |
| 0.10 | 97 |
| 0.20 | 194 |
| 0.40 | 423 |

As shown in Table 1 and Fig. 26, use of the device for measuring CRP of Example 1 revealed an excellent linearity in the range of CRP of 0.00 to 0.40 µg/mL.

### (2) Measurement of CRP concentration in human serum

Using a human serum as a sample in stead of the CRP standard solution, amount of alteration in the frequency was measured similarly to the case of production of the calibration curve in the paragraph (1), and the CRP concentration in the human serum was calculated with the calibration curve produced in the paragraph (1) leading to a result of 0.092 µg/mL. On the other hand, CRP concentration in the human serum was measured using Exeter CRP (manufactured by Kyowa Medex Co., Ltd.) that is a kit for measuring CRP leading to a result of 0.094 µg/mL. Therefore, it was revealed that use of the device for measuring CRP of Example 1 enables the CRP concentration in a human serum to be measured accurately.

### Comparative Example 1: Comparison of device for measuring CRP of Example 1 with conventional apparatus for immunochromatography with respect to measurement of CRP concentration in sample

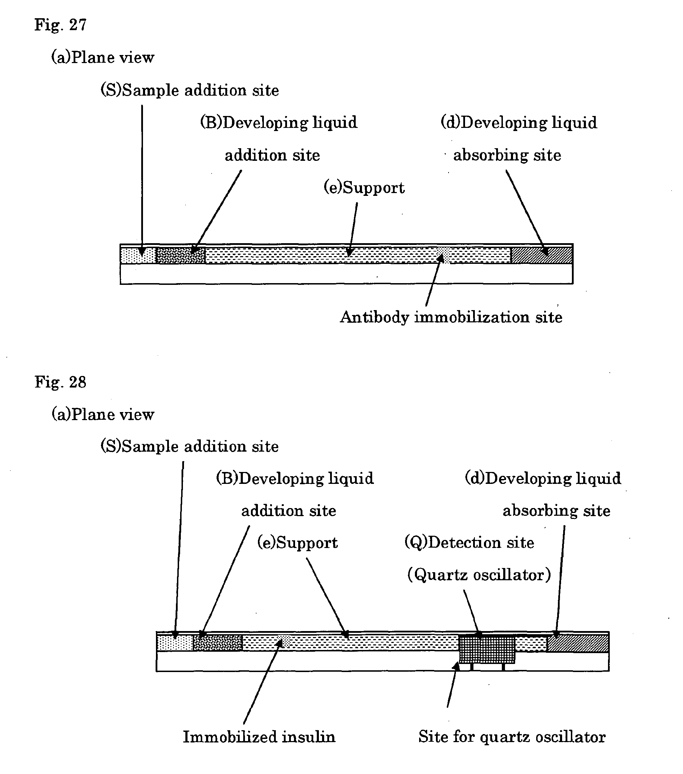

Using a conventional device for immunochromatography as shown in Fig. 27 which comprises a member for sample addition for adding a sample, a member for retaining labeled binder to which a microsphere-labeled anti-human CRP mouse monoclonal antibody (IgG) was bound, a support consisting of a nitrocellulose membrane (FF85), and a developing liquid absorbing member including an anti-human CRP goat polyclonal antibody (IgG) immobilization site and absorptive paper 300, detection limit of CRP in a sample was studied. As the sample, <1> 10 mmol/L tris buffer, pH 7.5 alone, <2> 0.05 µg/mL CRP standard solution, <3> 0.1 µg/mL CRP standard solution, <4> 0.2 µg/mL CRP standard solution, <5> 0.4 µg/mL CRP standard solution, and <6> 4.0 µg/mL CRP standard solution were used, respectively. As a result, blue line could be identified only in the case of the sample <6>. Therefore, it was revealed that the conventional apparatus for immunochromatography of the present invention can measure CRP in a sample with a higher sensitivity in comparison with the conventional apparatus for immunochromatography.

### Example 3: Device for measuring anti-tubercle bacilli surface glycolipid antigen antibody (anti-TBGL antibody)

A gold colloid-labeled anti-human immunoglobulin mouse monoclonal antibody (IgG) was diluted 100 times in a 10 mmol/L phosphate buffer, pH 7.4 that contains sodium chloride at a concentration of 0.15 mol/L, and this diluted gold colloid-labeled antibody solution was defined as a labeled solution. Absorptive paper 470 type impregnated with this labeled solution was vacuum dried at 3 mmHg or less to produce a member for retaining labeled binder.

A quartz oscillator was immersed in a 0.1 mg/mL solution of tubercle bacilli surface glycolipid antigen (TBGL) that contains trehalose dimycolate as a principal component in hexane to allow application. After vacuum drying at 3 mmHg or less, it was blocked with a 10 mmol/L phosphate buffer containing 1% BSA to produce a TBGL immobilized quartz oscillator.

Using the aforementioned member for retaining labeled binder and the TBGL immobilized quartz oscillator, a device for measuring an anti-TBGL antibody was produced according to a method that is similar to Example 1. Using the apparatus, the frequency in each sample was measured for a serum of a healthy person (not older than the age of 20), a serum of a tuberculous patient, and a cutoff standard solution of a determiner TBGL antibody (antibody titer: 2 unit; manufactured by Kyowa Medex Co., Ltd.) as a sample. Difference between the frequency prior to adding the sample and the measured frequency [amount of decrease in frequency (-ΔF/Hz)] was calculated, and additionally, titer of the anti-TBGL antibody included in the serum of the healthy person (not older than the age of 20), and titer of the anti-TBGL antibody included in the serum of the tuberculous patient were calculated from the frequency calculated as described above. The results are presented in Table 2.

**Table 2**

| | Amount of decrease in frequency (-ΔF/Hz) | Antibody titer | Assessment |
|---|---|---|---|
| Cut off standard solution | 2864 | 2 | - |
| Serum from healthy person | 890 | 0.3 | negative |
| Serum from tuberculous patient | 9317 | 3.2 | positive |

As shown in Table 2, in the serum of the healthy person, titer of the anti-TBGL antibody is not higher than the cutoff value, however, to the contrary, in the serum of the tuberculous patient, the titer of the anti-TBGL antibody was not lower than the cutoff value. Therefore, it was suggested that use of the device for measuring anti-TBGL of the present invention is applicable to diagnoses of tuberculosis.

### Example 4: Device for measuring insulin

### (1) Production of member for retaining labeled binder containing gold colloid-labeled anti-human insulin mouse monoclonal antibody (IgG)

An anti-human insulin mouse monoclonal antibody (IgG) was dissolved in a 0.1 mol/L borate buffer (pH 8.0) to prepare a 2.0 mg/mL anti-human insulin mouse monoclonal antibody (IgG) solution. To this solution (1.0 mL) was added a 1.0 mg/mL aqueous 2-iminothiolane solution (0.3 mL). After allowing the mixture to react at 30°C for 0.5 hour, thus resulting reaction mixture was fractionated on column chromatography with Sephadex G-25 [elution solvent: 0.1 mmol/L borate buffer (pH 8.0)] to obtain a 0.15 mg/mL sulfhydrylated anti-human insulin mouse monoclonal antibody (IgG) solution in borate buffer.

To this sulfhydrylated anti-human insulin mouse monoclonal antibody (IgG) solution (1 mL) in borate buffer were added 10 mL of gold colloidal particles having a particle size of 40 nm. The mixture was allowed to react at room temperature for 10 min, thereby preparing a solution containing the gold colloidal particle-labeled anti-human insulin mouse monoclonal antibody. To the solution was added a 10 mmol/L imidazole buffer, pH 7.8 (50 mL) containing 0.6% BSA. Further, the mixed solution was subjected to an ultrasonic treatment to give a labeled solution. After impregnation of the labeled solution to a nitrocellulose membrane [AccuFlow G type], the membrane was vacuum dried at room temperature, 3 mmHg or less for 1 hour to produce a member for retaining labeled binder having the gold colloidal particle-labeled anti-human insulin mouse monoclonal antibody retained.

### (2) Immobilization of insulin on nitrocellulose membrane

To a defined region on the nitrocellulose membrane (FF85) was added a 1% aqueous glutaraldehyde solution dropwise using BioJet Quanti 3000, and dried at room temperature for 0.5 hour. Thereafter, a 0.2 mg/mL aqueous insulin solution was added dropwise to the same region using BioJet Quanti 3000. After allowing the reaction at room temperature for 2 hours, a 1% BSA solution in PBS was added dropwise to the same region using BioJet Quanti 3000 to produce a support comprising insulin immobilized on a nitrocellulose membrane.

### (3) Immobilization of anti-mouse IgG goat polyclonal antibody on quartz oscillator

With a similar method of Example 1 (1), the anti-mouse IgG goat polyclonal antibody was sulfhydrylated, and immobilization of thus sulfhydrylated anti-mouse IgG goat polyclonal antibody on a gold electrode at the center of a quartz oscillator sensor chip (27 MHz) was carried out.

### (4) Production of device for measuring insulin

A strip for immunochromatography for measuring insulin as shown in Fig. 28 was produced by disposing the member for retaining labeled binder produced in the paragraph (1) containing the gold colloidal particle-labeled anti-human insulin mouse monoclonal antibody (IgG), the support produced in the paragraph (2) consisting of the nitrocellulose membrane on which insulin was immobilized, the anti-mouse IgG goat polyclonal antibody-immobilized quartz oscillator produced in the paragraph (3), and a developing liquid absorbing member consisting of the absorptive paper 300, thereby producing a device for measuring insulin.

### Example 5: Measurement of insulin in sample using device for measuring insulin

A sample (5 µL) was added dropwise to the member for sample addition. Next, a 10 mmol/L tris buffer, pH 7.5 was continuously added from the same pore. The buffer reached to the support to which the gold colloidal particle-labeled anti-human insulin mouse monoclonal antibody (IgG) was bound while diluting the sample, and at the site, insulin in the sample reacts with the gold colloidal particle-labeled anti-human insulin mouse monoclonal antibody (IgG). The reaction liquid further proceeded the membrane by the capillary flow, and the gold colloidal particle-labeled anti-human insulin mouse monoclonal antibody (IgG) which did not bind to insulin in the sample is captured at the insulin immobilization site. Following the reaction at the insulin immobilization part, a complex of insulin not immobilized on the insulin immobilization part and the gold colloidal particle-labeled anti-human insulin mouse monoclonal antibody (IgG) moved by the capillary flow onto the quartz oscillator, and filled in a fine space on the quartz oscillator. This complex is captured on the anti-mouse IgG goat polyclonal antibody-immobilized quartz oscillator, and the reaction liquid was absorbed to the absorptive pad. After completing absorption of the reaction liquid to the absorptive pad, the quartz oscillator was removed, and applied to AFFINIX Q (manufactured by Initium, Inc.). A 10 mmol/L tris buffer, pH 7.5 was added to the vessel, and the frequency was measured 5 min later.

As the sample, <1> 10 mmol/L tris buffer, pH 7.5, and <2> 300 ng/mL insulin standard solution were used, respectively. For each sample, the quartz oscillator was replaced to measure the frequency. First, the frequency prior to adding the sample was measured, and then, thereto was added <1> 10 mmol/L tris buffer, pH 7.5 (5 µL). The frequency after absorption of the buffer to the absorptive pad was measured, and difference between both frequencies was calculated (this difference in frequency being defined as Δ1). Next, the sample (5 µL) of <2> was added in stead of <1> 10 mmol/L tris buffer, pH 7.5 (5 µL), and similarly to the case of <1>, the frequency prior to adding each sample, and the frequency after absorption of the reaction liquid to the absorptive pad were measured to calculate the difference in both frequencies (difference in frequency corresponding to each sample being defined as Δ2). Value obtained by subtracting Δ1 from difference in frequency (Δ2), i.e., value obtained by subtracting blank was 1375 (-ΔF/Hz).

Next, similar measurement was carried out using a serum of a diabetic patient as a sample in stead of the insulin standard solution (300 ng/mL), revealing the value obtained by subtracting blank of 577 (-ΔF/Hz). From this value, the insulin concentration in the serum was calculated to be 126 ng/mL. On the other hand, the insulin concentration in the serum was measured using Chemilumi insulin (manufactured by Bayer Medical Ltd.) that is a kit for measuring insulin leading to a result of 125 ng/mL. Therefore, it was revealed that use of the device of Example 4 enables the insulin concentration in a human serum to be measured accurately.

### Example 6: Device for measuring CRP with kit for producing calibration curve

Six pieces of the device for measuring CRP produced in Example 1 were provided, and radially arranged as shown in Fig. 11. At each sample addition site in the vicinity of the center, for example, test liquid was added to the addition site of the device 1, and five standard solutions used in Example 2, i.e., <1> 10 mmol/L tris buffer, pH 7.5 alone, <2> 0.05 µg/mL CRP standard solution, <3> 0.1 µg/mL CRP standard solution, <4> 0.2 µg/mL CRP standard solution, and <5> 0.4 µg/mL CRP standard solution in an amount that is the same as the sample were added to the addition site of the devices 2 to 6, respectively. In a similar manner to the method in Example 2, a calibration curve was produced from the amount of alteration in the frequency obtained by subtracting the blank in the devices 2 to 6, and thus, the CRP concentration in the test liquid can be measured by applying the amount of alteration in the frequency obtained by subtracting the blank in the device 1.

### Industrial Applicability

According to the present invention, a device, an apparatus for measuring on immunochromatography and a method of the measurement which are simple and enable sensitively quantitative analyses are provided.

## Claims

1. A device for measuring an object to be measured in a sample which comprises a support, sample addition site (S) and a detection site (Q), said sample addition site and said detection site being on the support,
said support allowing the object to be measured to move by the capillary flow of a developing liquid,
said detection site having a piezoelectric vibrator sandwiched between two electrodes,
said piezoelectric vibrator having a trapper A (c1) immobilized thereon, or an analogue of the object to be measured (c1') immobilized thereon, and
said support further comprising a binder retaining site(BR) where a binder (b1) is retained therein so that it is movable by the capillary flow of the developing liquid.

2. The device according to claim 1, wherein the binder retaining site is provided between the sample addition site and the detection site.

3. The device according to claim 1, wherein the binder retaining site is provided on the opposite side of the sample addition site with the detection site being interposed therebetween.

4. A device for measuring an object to be measured in a sample which comprises a support, sample addition site (S) and a detection site (Q), said sample addition site and said detection site being on the support,
said support allowing the object to be measured to move by the capillary flow of a developing liquid,
said detection site having a piezoelectric vibrator sandwiched between two electrodes,
said piezoelectric vibrator having a trapper A (c1) immobilized thereon that binds to the object to be measured, or a trapper B (c3) immobilized thereon that binds to a complex of a binder with a labeled analogue of the object to be measured or the object to be measured and to a complex of a labeled binder with the object to be measured or the analogue of the object to be measured,
said support further comprising a binder retaining site (BR) where the binder (b1) is retained so that it is movable by the capillary flow of the developing liquid, and
said support further comprising an analogue of the object to be measured immobilizing site (DF) where the analogue of the object to be measured (b5) is immobilized between the binder retaining site and the detection site so that it is not moved by the capillary flow of the developing liquid, or an analogue of the object to be measured retaining site (DR) where the analogue of the object to be measured (b5) is retained on the support so that it is movable by the capillary flow of the developing liquid.

5. The device according to any one of claims 1 to 4, wherein the binder is labeled.

6. A device for measuring an object to be measured in a sample which comprises a support, sample addition site (S) and a detection site (Q), said sample addition site and said detection site being on the support,
said support allowing the object to be measured to move by the capillary flow of a developing liquid,
said detection site having a piezoelectric vibrator sandwiched between two electrodes, the piezoelectric vibrator having a binder (c2) immobilized thereon, and
said support further comprising an analogue of the object to be measured retaining site (DR) where the analogue of the object to be measured (b2) is retained so that it is movable by the capillary flow of the developing liquid.

7. The device according to claim 6, wherein the analogue of the object to be measured retaining site is provided between the sample addition site and the detection site.

8. The device according to claim 6, wherein the analogue of the object to be measured retaining site is provided on the opposite side of the sample addition site with the detection site being interposed therebetween.

9. A device for measuring an object to be measured in a sample which comprises a support, sample addition site (S) and a detection site (Q), said sample addition site and said detection site being on the support,
said support allowing the object to be measured to move by the capillary flow of a developing liquid,
said detection site having a piezoelectric vibrator sandwiched between two electrodes, the piezoelectric vibrator having a trapper B (c3) immobilized thereon, and
said support further comprising an analogue of the object to be measured retaining site (DR) where an analogue of the object to be measured (b2) is retained so that it is movable by the capillary flow of the developing liquid, and a binder retaining site (BR) where a binder (b3) is retained so that it is movable by the capillary flow of the developing liquid.

10. A device for measuring an object to be measured in a sample which comprises a support, sample addition site (S) and a detection site (Q), said sample addition site and said detection site being on the support,
said support allowing the object to be measured to move by the capillary flow of a developing liquid,
said detection site having a piezoelectric vibrator sandwiched between two electrodes, the piezoelectric vibrator having a binder (c2) immobilized thereon that binds to the object to be measured,
said support further comprising an analogue of the object to be measured retaining site (DR) where an analogue of the object to be measured (b2) is retained so that it is movable by the capillary flow of the developing liquid, and
said support further comprising a binder immobilizing site (BF) where a binder (b4) is immobilized on the support between the analogue of the object to be measured retaining site and the detection site so that it is not movable by the capillary flow of the developing liquid.

11. The device according to any one of claims 6 to 10, wherein the analogue of the object to be measured is labeled.

12. The device according to any one of claims 5 or 11, wherein the label is an insoluble particle.

13. The device according to claim 12, wherein the insoluble particle is a metal colloid or latex.

14. A device for measuring an object to be measured in a sample which comprises a support, sample addition site (S) and a detection site (Q), said sample addition site and said detection site being on the support,
said support allowing the object to be measured to move by the capillary flow of a developing liquid,
said detection site having a piezoelectric vibrator sandwiched between two electrodes, and
said piezoelectric vibrator having a trapper A (c1), an analogue of the object to be measured (c1'), a binder (c2) or a trapper B (c3).

15. The device according to any one of claims 1 to 14, which further comprises a developer absorbing site (d).

16. The device according to any one of claims 1 to 15, wherein the detection site has another piezoelectric vibrator which is sandwiched between two electrodes and on which none of the trapper A (c1), the analogue of the object to be measured (c1'), the binder (c2) and the trapper B (c3) are immobilized, in addition to the piezoelectric vibrator which is sandwiched between two electrodes and on which the trapper A (c1), the analogue of the object to be measured (c1'), the binder (c2) or the trapper B (c3) is immobilized.

17. The device according to any one of claims 1 to 16, wherein the piezoelectric vibrator is a quartz oscillator.

18. An apparatus for measuring an object to be measured which comprises the device according to any one of claims 1 to 17, a frequency measuring circuit which measures the frequency of vibration of the piezoelectric vibrator and which is connected to the electrode of the piezoelectric vibrator of the device, and a concentration arithmetic circuit which calculates a concentration of the object to be measured based on the frequency and which is connected to the frequency measuring circuit.

19. A method for quantitatively determining an object to be measured in a sample which comprises the steps of:
preparing a piezoelectric vibrator having a substance immobilized thereon, said substance being a trapper A (c1) or an analogue of the object to be measured (c1'),
supplying the sample and a binder to the substance through a support where the object to be measured and the binder are movable by the capillary flow of the developing liquid,
allowing the object to be measured in the sample to bind specifically to the substance immobilized on the piezoelectric vibrator, and
quantitatively determining alteration in frequency of a piezoelectric vibrator generated by the specific binding with the substance immobilized on the piezoelectric vibrator.

20. The method according to claim 19, wherein the sample and the binder are supplied by the capillary flow in the same direction toward the trapper A or the analogue of the object to be measured.

21. The method according to claim 19, wherein the sample and the binder are supplied by the capillary flow in the reverse direction with respect to the trapper A or the analogue of the object to be measured.

22. A method for quantitatively determining an object to be measured in a sample which comprises the steps of:
preparing a piezoelectric vibrator having a substance immobilized thereon, said substance being a trapper A (c1) or a trapper B (c3),
supplying the sample and a binder to the substance through a support where the object to be measured and the binder are movable by the capillary flow of the developing liquid, but where an analogue of the object to be measured (b5) is immobilized so that it is not movable by the capillary flow of the developing liquid,
allowing the object to be measured in the sample to bind specifically to the substance immobilized on the piezoelectric vibrator, and
quantitatively determining alteration in frequency of a piezoelectric vibrator generated by the specific binding with the substance immobilized on the piezoelectric vibrator.

23. A method for quantitatively determining an object to be measured in a sample which comprises the steps of:
preparing a piezoelectric vibrator having a substance immobilized thereon, said substance being a trapper A (c1) or an analogue of the object to be measured (c1'),
supplying the sample and a binder to the substance through a support where the object to be measured and the binder are movable by the capillary flow of the developing liquid,
allowing the object to be measured in the sample to bind specifically to the substance immobilized on the piezoelectric vibrator, and
quantitatively determining alteration in frequency of a piezoelectric vibrator generated by the specific binding with the substance immobilized on the piezoelectric vibrator.

24. The method according to any one of claims 17 to 23, wherein the binder is labeled.

25. A method for quantitatively determining an object to be measured in a sample which comprises the steps of:
preparing a piezoelectric vibrator having a substance immobilized thereon, said substance being a binder (c2),
supplying the sample and an analogue of the object to be measured to the substance through a support where the object to be measured and the analogue of the object to be measured are movable by the capillary flow of the developing liquid,
allowing the object to be measured in the sample to bind specifically to the substance immobilized on the piezoelectric vibrator, and
quantitatively determining alteration in frequency of a piezoelectric vibrator generated by the specific binding with the substance immobilized on the piezoelectric vibrator.

26. The method according to claim 25, wherein the sample and the analogue of the object to be measured are supplied by the capillary flow in the same direction toward the binder.

27. The method according to claim 25, wherein the sample and the analogue of the object to be measured are supplied by the capillary flow in the reverse direction with respect to the binder.

28. A method for quantitatively determining an object to be measured in a sample which comprises the steps of:
preparing a piezoelectric vibrator having a substance immobilized thereon, said substance being a trapper B (c3),
supplying the sample, an analogue of the object to be measured (b2) and a binder (c3) to the substance through a support where the object to be measured, the analogue of the object to be measured (b2) and the binder (c3) are movable by the capillary flow of the developing liquid,
allowing the object to be measured in the sample to bind specifically to the substance immobilized on the piezoelectric vibrator, and
quantitatively determining alteration in frequency of a piezoelectric vibrator generated by the specific binding with the substance immobilized on the piezoelectric vibrator.

29. A method for quantitatively determining an object to be measured in a sample which comprises the steps of:
preparing a piezoelectric vibrator having a substance immobilized thereon, said substance being a binder (c2),
supplying the sample and an analogue of the object to be measured to the substance through a support where the object to be measured and the analogue of the object to be measured are movable by the capillary flow of the developing liquid, but where a binder (b4) is immobilized so that it is not movable by the capillary flow of the developing liquid,
allowing the object to be measured in the sample to bind specifically to the substance immobilized on the piezoelectric vibrator, and
quantitatively determining alteration in frequency of a piezoelectric vibrator generated by the specific binding with the substance immobilized on the piezoelectric vibrator.

30. The method according to any one of claims 25 to 29, wherein the analogue of the object to be measured is a labeled substance.

31. The method according to claims 24 or 30, wherein the label is an insoluble particle.

32. The method according to claim 31, wherein the insoluble particle is a metal colloid or latex.

33. A method for quantitatively determining an object to be measured in a sample which comprises the steps of:
preparing a piezoelectric vibrator having a substance immobilized thereon, said substance being a trapper A (c1),
supplying the sample to the substance through a support where the object to be measured is movable by the capillary flow of the developing liquid,
allowing the object to be measured in the sample to bind specifically to the substance immobilized on the piezoelectric vibrator, and
quantitatively determining alteration in frequency of a piezoelectric vibrator generated by the specific binding with the substance immobilized on the piezoelectric vibrator.

34. The method according to any one of claims 17 to 29, wherein a second piezoelectric vibrator on which none of the trapper A (c1), the analogue of the object to be measured (c1'), the binder (c2) and the trapper B (c3) are immobilized is used, the method further comprising supplying the sample to a first piezoelectric vibrator on which the trapper A (c1), the analogue of the object to be measured (c1'), the binder (c2) or the trapper B (c3) is immobilized and to the second piezoelectric vibrator, and using the second frequency as a control.

35. The method according to any one of claims 19 to 34, wherein the piezoelectric vibrator is a quartz oscillator.
